(19) Europäisches Patentamt
European Patent Office
Office européen des brevets

(11) EP 3 558 927 B1

(12) **EUROPÄISCHE PATENTSCHRIFT**

(45) Veröffentlichungstag und Bekanntmachung des Hinweises auf die Patenterteilung:
**20.01.2021 Patentblatt 2021/03**

(21) Anmeldenummer: **17816842.3**

(22) Anmeldetag: **18.12.2017**

(51) Int Cl.:
***C07C 209/78*** *(2006.01)* ***C07C 209/54*** *(2006.01)*
***C07C 209/60*** *(2006.01)* ***C07C 211/48*** *(2006.01)*
***C07C 211/50*** *(2006.01)*

(86) Internationale Anmeldenummer:
**PCT/EP2017/083264**

(87) Internationale Veröffentlichungsnummer:
**WO 2018/114771 (28.06.2018 Gazette 2018/26)**

(54) **VERFAHREN ZUR HERSTELLUNG VON DI- UND POLYAMINEN DER DIPHENYLMETHANREIHE**

PROCESS FOR THE PREPARATION OF DI- AND POLYAMINES OF THE DIPHENYLMETHANE SERIES

PROCÉDÉ DE PRÉPARATION DE DIAMINES ET POLYAMINES DE LA SÉRIE DES DIPHÉNYLMÉTHANES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2016 EP 16206116**

(43) Veröffentlichungstag der Anmeldung:
**30.10.2019 Patentblatt 2019/44**

(73) Patentinhaber: **Covestro Intellectual Property GmbH & Co. KG**
**51373 Leverkusen (DE)**

(72) Erfinder:
- **KNAUF, Thomas**
**41542 Dormagen (DE)**
- **WERSHOFEN, Stefan**
**41065 Mönchengladbach (DE)**
- **GRUNER, Klaus-Gerd**
**47239 Duisburg (DE)**
- **HARTJES, Volker**
**47239 Duisburg (DE)**

(74) Vertreter: **Levpat**
**c/o Covestro AG**
**Gebäude 4825**
**51365 Leverkusen (DE)**

(56) Entgegenhaltungen:
**EP-A1- 1 616 890**
**WO-A1-2014/173856**
**WO-A1-2015/197519**
**WO-A1-2015/197520**
**WO-A1-2015/197527**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 558 927 B1

## Beschreibung

Die vorliegende Erfindung betrifft ein Verfahren zur Herstellung von Di- und Polyaminen der Diphenylmethanreihe durch Kondensation von Anilin und Formaldehyd gefolgt von säurekatalysierter Umlagerung bei unterschiedlichen Produktionskapazitäten unter Veränderung des Gehalts an *Di*aminen der Diphenylmethanreihe (Änderung des *Zweikerngehalts*). Durch Anpassung des molaren Verhältnisses von insgesamt eingesetztem Anilin zu insgesamt eingesetztem Formaldehyd und der Reaktionstemperatur wird erreicht, dass trotz der mit der veränderten Produktionskapazität unweigerlich verbundenen Änderung der Verweilzeit die Umlagerungsreaktion vollständig abläuft und die Bildung unerwünschter Nebenprodukte weitestgehend vermieden sowie die angestrebte Änderung des Zweikerngehalts erzielt wird.

Die Herstellung von Di- und Polyaminen der Diphenylmethanreihe (MDA) durch Umsetzung von Anilin mit Formaldehyd in Gegenwart saurer Katalysatoren ist grundsätzlich bekannt. Im Sinne der vorliegenden Erfindung werden unter Di- und Polyaminen der Diphenylmethanreihe Amine und Gemische von Aminen folgenden Typs verstanden:

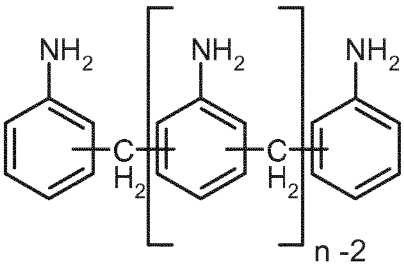

(I)

Dabei steht n für eine natürliche Zahl ≥ 2. Im Folgenden werden die Verbindungen dieses Typs, bei denen n = 2 ist, als *Di*amine der Diphenylmethanreihe oder Diaminodiphenylmethane (nachfolgend MMDA; "Monomer-MDA") bezeichnet. Verbindungen dieses Typs, bei denen n > 2 ist, werden im Rahmen dieser Erfindung als *Poly*amine der Diphenylmethanreihe oder Polyphenylenpolymethylenpolyamine (nachfolgend PMDA; "Polymer-MDA") bezeichnet. Gemische aus beiden Typen werden als Di- *und* Polyamine der Diphenylmethanreihe bezeichnet (der Einfachheit halber nachfolgend als MDA bezeichnet). Die korrespondierenden Isocyanate, die sich formal durch Ersatz aller $NH_2$-Gruppen durch NCO-Gruppen aus den Verbindungen der Formel (I) ableiten lassen, werden dementsprechend als Diisocyanate der Diphenylmethanreihe (nachfolgend MMDI), Polyisocyanate der Diphenylmethanreihe oder Polyphenylenpolymethylenpolyisocyanate (nachfolgend PMDI) bzw. Di- und Polyisocyanate der Diphenylmethanreihe (nachfolgend MDI) bezeichnet. Die höheren Homologen (n > 2) liegen dabei sowohl beim Amin als auch beim Isocyanat in der Regel immer im Gemisch mit den Dimeren (n = 2) vor, so dass in der Praxis nur zwei Produkttypen relevant sind, die reinen Dimeren (MMDA bzw. MMDI) und das Gemisch aus Dimeren und höheren Homologen (MDA bzw. MDI). Die Stellung der Amingruppen an den beiden Phenylengruppen der Dimeren (*para-para*; *ortho-para* und *ortho-ortho*) wird im Folgenden nur angegeben, wenn es von Belang ist. Der Einfachheit halber geschieht dies in der Form X,Y'-MDA (4,4'-, 2,4'- bzw. 2,2'-MDA), wie in der Literatur üblich. Für MDI gilt Entsprechendes (Kennzeichnung der Isomere als X,Y'-MDI (4,4'-, 2,4'- bzw. 2,2'-MDI).

Industriell werden die Di- und Polyamingemische überwiegend durch Phosgenierung zu den entsprechenden Di- und Polyisocyanaten der Diphenylmethanreihe umgesetzt.

Die kontinuierliche oder teilweise diskontinuierliche Herstellung von MDA ist z. B. in EP-A-1 616 890, US 5,286,760, EP-A-0 451 442 und WO-A-99/40059 offenbart. Die saure Kondensation von aromatischen Aminen und Formaldehyd zu Di- und Polyaminen der Diphenylmethanreihe läuft in mehreren Reaktionsschritten ab. Beim sog. "Aminalverfahren" werden zunächst in Abwesenheit eines sauren Katalysators Formaldehyd mit Anilin zu sogenanntem Aminal kondensiert, wobei Wasser abgespalten wird. Danach erfolgt die säurekatalysierte Umlagerung zum MDA in einem ersten Schritt zu para- bzw. ortho-Aminobenzylanilin. Die Aminobenzylaniline lagern in einem zweiten Schritt zu MDA um. Hauptprodukte der säurekatalysierten Reaktion von Anilin und Formaldehyd sind das Diamin 4,4'-MDA, seine Stellungsisomere 2,4'-MDA und 2,2'-MDA sowie die höheren Homologen (PMDA) der verschiedenen Diamine. Beim sog. "Neutralisationsverfahren" werden Anilin und Formaldehyd in Anwesenheit eines sauren Katalysators direkt zu Aminobenzylanilinen umgesetzt, die anschließend weiter zu den zweikernigen MMDA-Isomeren und höherkernigen PMDA-Homologen umlagern. Die vorliegende Erfindung betrifft das Aminalverfahren.

Unabhängig von der Verfahrensvariante zur Herstellung des sauren Reaktionsgemisches wird dessen Aufarbeitung gemäß dem Stand der Technik durch Neutralisation mit einer Base eingeleitet. Diese Neutralisation erfolgt üblicherweise bei Temperaturen von beispielsweise 90 °C bis 100 °C ohne Zusatz weiterer Substanzen (vgl. H. J. Twitchett, Chem. Soc. Rev. 3(2), 223 (1974)). Sie kann aber auch auf einem anderen Temperaturniveau erfolgen, um

z. B. den Abbau von störenden Nebenprodukten zu beschleunigen. Hydroxide der Alkali- und Erdalkalielemente sind als Basen geeignet. Vorzugsweise kommt Natronlauge zum Einsatz.

Im Anschluss an die Neutralisation wird die organische von der wässrigen Phase in einem Trennbehälter getrennt. Die nach Abtrennung der wässrigen Phase verbleibende, Roh-MDA enthaltende organische Phase wird weiteren Aufarbeitungsschritten unterzogen, wie z. B. einer Wäsche mit Wasser (Basenwäsche), um Restsalze aus dem Roh-MDA zu waschen. Abschließend wird das so gereinigte Roh-MDA von überschüssigem Anilin, Wasser und anderen im Gemisch vorhandenen Stoffen (z. B. Lösungsmittel) durch geeignete Verfahren wie z. B. Destillation, Extraktion oder Kristallisation befreit. Die nach dem Stand der Technik übliche Aufarbeitung ist beispielsweise offenbart in EP-A-1 652 835, Seite 3, Zeile 58 bis Seite 4, Zeile 13 oder EP-A-2 103 595, Seite 5, Zeile 21 bis 37.

Gegenstand der internationalen Patentanmeldung WO 2014/173856 A1 ist ein Verfahren zur Herstellung von Di- und Polyaminen der Diphenylmethanreihe durch Umsetzung von Anilin und Formaldehyd in Abwesenheit eines Säurekatalysators zu Aminal und Wasser, Abtrennung der wässrigen Phase und Weiterverarbeitung der organischen Aminalphase zu den Di- und Polyaminen der Diphenylmethanreihe, bei dem durch Einsatz eines Koaleszenz-Hilfsmittels in der Phasentrennung des in Aminalreaktion erhaltenen Verfahrensprodukts der Anteil an Wasser und damit auch an wasserlöslichen Verunreinigungen in der organischen, das Aminal enthaltenden Phase verringert wird. Die nach Weiterverarbeitung der Aminalphase durch säurekatalysierte Umlagerung und Aufarbeitung gewonnenen Di- und Polyamine der Diphenylmethanreihe eignen sich hervorragend zur Herstellung der entsprechenden Isocyanate.

Die Güte eines Reaktionsverfahrens zur Herstellung von MDA ist einerseits definiert durch den Gehalt des Roh-Produktes an unerwünschten Nebenkomponenten und Verunreinigungen, die durch unsachgemäße Reaktionsführung entstehen können. Andererseits ist die Güte eines Reaktionsverfahrens dadurch definiert, dass der gesamte Prozess ohne technischen Produktionsausfall oder Probleme, die zu einem Eingriff in den Prozess nötigen, betrieben werden kann und dass Verluste an Einsatzstoffen vermieden oder zumindest minimal gehalten werden.

Den beschriebenen Verfahren des Standes der Technik gelingt es zwar, mit hoher Ausbeute MDA herzustellen, ohne dass es bei den Endprodukten zu einer Qualitätseinbuße kommt, jedoch werden nur Verfahren beschrieben, die sich im Normalbetrieb befinden. Nur wenige Veröffentlichungen befassen sich mit Zuständen außerhalb des Normalbetriebs:

Die internationale Patentanmeldung WO 2015/197527 A1 betrifft ein Verfahren zur Herstellung von Di- und Polyaminen der Diphenylmethanreihe (MDA), eine Anlage zur Herstellung von MDA und ein Verfahren zum Betrieb einer Anlage zur Herstellung von MDA. Die Erfindung ermöglicht es, Produktionsstillstände beim Betreiben des MDA-Prozesses hinsichtlich Zeitaufwand und gegebenenfalls auch hinsichtlich Energie- und Materialverbrauch durch eine sog. Kreislauffahrweise einzelner Anlagenteile zu optimieren. Während einer Unterbrechung des Verfahrens bzw. Unterbrechung des Betriebs einzelner Anlagenteile findet kein Eintrag von Formaldehyd in die Reaktion statt, und die nicht von einer Revisions-, Reparatur- oder Reinigungsmaßnahme betroffenen Anlagenteile werden in sog. Kreislauffahrweise betrieben. Dadurch wird unter anderem erreicht, dass nur das betroffene Anlagenteil für die Zeit der Maßnahme stillgelegt werden muss, was vorteilhaft hinsichtlich Produktivität und Wirtschaftlichkeit des Verfahrens sowie der Qualität der hergestellten Produkte sein kann.

Die internationale Patentanmeldung WO 2015/197519 A1 betrifft ein Verfahren zur Herstellung von Di- und Polyaminen der Diphenylmethanreihe, bei dem während des Abfahrvorganges des Produktionsprozesses dafür Sorge getragen wird, dass ein Überschuss von Anilin gegenüber Formalin gewährleistet ist.

Die internationale Patentanmeldung WO 2015/197520 A1 betrifft ein Verfahren zur Herstellung von Di- und Polyaminen der Diphenylmethanreihe (MDA) aus Anilin und Formaldehyd, bei dem während des Anfahrvorganges Sorge getragen wird, dass ein ausreichender Überschuss von Anilin gegenüber Formaldehyd gewährleistet ist, der mindestens 105 % des für die Zielrezeptur des zu produzierenden MDA angestrebten molaren Verhältnisses von Anilin zu Formaldehyd beträgt.

Änderungen der angestrebten Produktionskapazität (auch "Lastwechsel" genannt) während der kontinuierlichen Produktion (also mit einem Anfangs- und Endzustand, in dem MDA produziert wird) werden auch hier nicht berücksichtigt. Da Anilin üblicherweise im stöchiometrischen Überschuss eingesetzt wird, ist die Produktionskapazität einer gegebenen Anlage zur Herstellung von MDA durch die Formaldehyd-Zufuhr definiert, die im Rahmen dieser Erfindung als (Formaldehyd-)Last bezeichnet wird.

Lastwechsel sind immer wiederkehrende Anlagenzustände, die einen erheblichen Einfluss auf das wirtschaftliche (und umweltschonende - Stichwort: Energieverbrauch) Betreiben einer kontinuierlich arbeitenden Anlage haben. Neben den bisher erwähnten "beabsichtigten" Lastwechseln (z. B. Lastreduktion als Folge gesunkener Nachfrage), gibt es auch Lastwechsel, die sich als Folge einer gewünschten Änderung der Produktzusammensetzung *zwangsläufig* ergeben. Dies ist etwa dann der Fall, wenn zur Erzielung einer angestrebten Änderung der Produktzusammensetzung das Verhältnis von insgesamt eingesetztem Anilin zu insgesamt eingesetztem Formaldehyd (in der Literatur auch als A/F-Verhältnis bezeichnet) signifikant erhöht werden soll, also - bei gleicher Formaldehydmenge - signifikant mehr Anilin in den Reaktor eingetragen werden müsste. Da die Abmessungen des Reaktors feststehen, kann jedoch die Anilinmenge nicht beliebig gesteigert werden. Um das gewünschte A/F-Verhältnis zu erreichen, kann es daher erforderlich werden,

die Formaldehydzufuhr und damit die Produktionskapazität (die "Last") zu reduzieren. Soll das A/F-Verhältnis signifikant erniedrigt werden und dies durch eine Erhöhung der Formaldehydzufuhr - bei gleichbleibender Anilinzufuhr - bewirkt werden, ist damit zwangsläufig ein Lastwechsel verbunden. Bei dieser Art von Lastwechseln bestehen besondere betriebstechnische Herausforderungen, da *zwei für den Betrieb einer Produktionsanlage bedeutsame Faktoren* - nämlich zum einen der Lastwechsel selbst, der bei kontinuierlicher Produktion zwangsläufig mit einer *Änderung der Verweilzeit* verbunden ist, und zum anderen die angestrebte Änderung der Produktzusammensetzung, die auch ohne Lastwechsel *Änderungen der Reaktionsparameter* erfordern würde - *gleichzeitig zusammenkommen.* Dies gilt natürlich auch dann, wenn ein Lastwechsel bei beabsichtigter Änderung der Produktzusammensetzung nicht zwangsläufig erforderlich wird, sondern aus anderen Gründen sogar gewünscht ist (beispielsweise weil eine Nachfrage nach einem Produkt anderer Zusammensetzung in - im Vergleich zum Produkt in der üblichen Zusammensetzung - geringerer oder höherer Menge besteht).

Die Produktzusammensetzung eines Gemisches aus Di- und Polyaminen der Diphenylmethanreihe ist insbesondere durch zwei Faktoren gekennzeichnet, nämlich:

1. durch den auf die Gesamtmasse an Di- und Polyaminen der Diphenylmethanreihe (MDA) bezogenen Massenanteil des *Di*amins der Diphenylmethanreihe (MMDA), fortan $\omega_{MMDA}$, ("Zweikerngehalt") und

2. durch die Isomerenverteilung *innerhalb* des MMDA-Anteils, welche - da 2,2'-MDA meist nur in untergeordneten Anteilen vorliegt - zweckmäßigerweise durch den auf die Gesamtmasse an Diaminen der Diphenylmethanreihe (auch als *Methylendiphenylendiamine* bezeichnet) bezogenen Massenanteil an 2,4'-Methylendiphenylendiamin ($\omega_{2,4'\text{-}MDA}$,) beschrieben wird.

Die vorliegende Erfindung befasst sich mit einer **Änderung des ersten Faktors.** Die Bestimmung des Massenanteils an Diaminen der Diphenylmethanreihe im MDA erfolgt üblicherweise durch Hochleistungsflüssigkeitschromatographie (HPLC), wovon auch im Rahmen der vorliegenden Erfindung bevorzugt Gebrauch gemacht wird.

Es wäre wünschenswert, ein Verfahren zur Herstellung von Di- und Polyaminen der Diphenylmethanreihe zur Verfügung zu haben, bei dem es durch einfache Maßnahmen gelingt, eine Änderung des Zweikerngehalts in Verbindung mit einem Lastwechsel so auszugestalten, dass das Verfahren in wirtschaftlicher, ökologischer und betriebstechnischer Hinsicht möglichst optimal (etwa hinsichtlich Ausbeute, Zeitaufwand, Energieverbrauch und Vermeidung von verfahrenstechnischen Problemen wie Anbackungen oder Verstopfungen in Apparaten) weiter betrieben werden kann.

Erfindungsgemäß kann dem durch ein **Verfahren zur Herstellung von Di- und Polyaminen gemäß Patentanspruch 1** Rechnung getragen werden. Dieses Verfahren ist, wie im Folgenden noch näher erläutert wird, insbesondere dadurch gekennzeichnet, dass bei einer angestrebten Laststeigerung die Temperatur in mindestens einem Umlagerungsreaktor erhöht und bei einer angestrebten Lastsenkung erniedrigt wird sowie dadurch, dass bei einer angestrebten Erhöhung des Zweikerngehalts das molare Verhältnis von insgesamt eingesetztem Anilin zu insgesamt eingesetztem Formaldehyd erhöht und bei einer angestrebten Erniedrigung des Zweikerngehalts gesenkt wird.

Alle Ausgestaltungen der vorliegenden Erfindung betreffen ein **Verfahren zur Herstellung von Di- und Polyaminen der Diphenylmethanreihe aus Anilin (1) und Formaldehyd (2)** in einer Produktionsanlage (10 000), wobei das molare Verhältnis von insgesamt eingesetztem Anilin (1) zu insgesamt eingesetztem Formaldehyd (2), n(1)/n(2), stets gleich oder größer als 1,6 ist, umfassend die Schritte:

(A-I) **Umsetzung von Anilin (1) und Formaldehyd (2) in Abwesenheit eines sauren Katalysators** in einem Reaktor (1000, Aminalreaktor) unter Erhalt eines Aminal (3) enthaltenden Reaktionsgemisches (4), und anschließende mindestens teilweise Abtrennung einer wässrigen Phase (6) aus dem Reaktionsgemisch (4) in einer Abtrenneinrichtung (2000, auch Aminalabscheider genannt) unter Erhalt einer organischen, das Aminal (3) enthaltenden Phase (5);

(A-II) Inkontaktbringen der in Schritt (A-I) erhaltenen organischen, das Aminal enthaltenden Phase (5) mit einem sauren Katalysator (7) in einer Reaktorkaskade (3000) aus i in Serie geschalteten Reaktoren j (3000-j = 1, 3000-j = 2, ..., 3000-j = i; fortan kurz 3000-1, 3000-2, ..., 3000-i), wobei i eine natürliche Zahl von 2 bis 10 ist **(säurekatalysierte Umlagerung),** wobei

- der in Strömungsrichtung erste Reaktor (3000-1) bei einer Temperatur $T_{3000\text{-}1}$ im Bereich von 25,0 °C bis 65,0 °C, bevorzugt im Bereich von 30,0 °C bis 60,0 °C, betrieben wird und mit dem Strom (5) und saurem Katalysator (7) sowie optional mit weiterem Anilin (1) und/oder weiterem Formaldehyd (2) beschickt wird,

- jeder in Strömungsrichtung nachfolgende Reaktor (3000-2, ..., 3000-i) bei einer Temperatur von mehr als 2,0 °C oberhalb von $T_{3000\text{-}1}$ betrieben wird und mit dem im unmittelbar vorangehenden Reaktor erhaltenen Reaktionsgemisch beschickt wird;

(B) **Isolierung der Di- und Polyamine der Diphenylmethanreihe** aus dem im letzten Reaktor (3000-i) aus Schritt (A-II) erhaltenen Reaktionsgemisch (8-i) durch mindestens

(B-I) Zugabe eines bezogen auf die insgesamt eingesetzte Menge an saurem Katalysator (7) stöchiometrischen Überschusses an Base (9) zu dem in Schritt (A-II) im letzten Reaktor (3000-i) erhaltenen Reaktionsgemisches (8-i) in einer Neutralisationseinrichtung (4000, bevorzugt Neutralisationsrührbehälter) unter Erhalt eines Reaktionsgemisches (10), wobei insbesondere ein molares Verhältnis von Base (9) zu insgesamt eingesetztem saurem Katalysator (7) im Bereich von 1,01 bis 1,30 eingehalten wird **(Neutralisation);**

(B-II) **Auftrennen** des in Schritt (B-I) erhaltenen Reaktionsgemisches (10) in einer Auftrenneinrichtung (5000, Neutralisationsabscheider) in eine organische Phase (11), umfassend Di- und Polyamine der Diphenylmethanreihe und eine wässrige Phase (12).

Die Ausdrücke *"insgesamt eingesetztes Anilin"* und *"insgesamt eingesetzter Formaldehyd"* bezeichnen jeweils die Gesamtmenge dieser im erfindungsgemäßen Verfahren eingesetzten Reaktanden, d. h. inklusive ggf. *nach* Schritt (A-I) zusätzlich zugesetzter Anteile.

Die organische Phase (11) kann, wie weiter unten noch näher beschrieben, weiter aufgearbeitet werden.

Erfindungsgemäß werden die oben genannten Schritte kontinuierlich durchgeführt, d. h. die jeweiligen Reaktanden werden dem dem jeweiligen Schritt zugeordneten Apparat kontinuierlich zugeführt und die Produkte werden demselben kontinuierlich entnommen.

Erfindungsgemäß wird nun so verfahren, dass man bei einem Lastwechsel die Temperatur in mindestens einem der Reaktoren 3000-2, ..., 3000-i *und* das molare Verhältnis von insgesamt eingesetztem Anilin zu insgesamt eingesetztem Formaldehyd (fortan n(1)/n(2)) in der weiter unten noch im Detail zu schildernden Art und Weise anpasst. Hierdurch wird erreicht, dass trotz veränderter Verweilzeit die Umlagerungsreaktion vollständig abläuft und vermehrte Nebenproduktbildung als Folge des Lastwechsels weitgehend vermieden wird, und zwar unter Erreichung der angestrebten Änderung des Zweikerngehalts. Eine Anpassung der Temperatur im in Strömungsrichtung ersten Reaktor (3000-1) ist nicht unbedingt erforderlich; jedoch werden im Falle dieses Reaktors *während* des Lastwechsels geringfügig größere Abweichungen von der Temperatur vor dem Lastwechsel zugelassen. Diese geringfügig größeren Toleranzen bei der Temperatur während des Lastwechsels erlauben es, den Lastwechsel regelungstechnisch einfacher beherrschen zu können. Die erfindungsgemäß zugelassenen Toleranzen, die weiter unten noch näher erläutert werden, sind jedoch noch in einem solchen Rahmen, dass betriebstechnische Probleme weitgehend bis vollständig vermieden werden.

Diese Vorgehensweise erlaubt es, Lastwechsel in Verbindung mit einer gewünschten Änderung des Zweikerngehalts in wirtschaftlicher, ökologischer (Stichwort: Energieverbrauch) und betriebstechnischer Hinsicht vorteilhaft auszugestalten.

Da die Erfindung Lastwechsel betrifft, also *verschiedene Betriebszustände einer Produktionsanlage,* sind folgende Begriffsdefinitionen hilfreich, welche im Folgenden verwendet werden:

**Ausgangszustand *A*** der Produktionsanlage mit gegebener, durch die Menge des eingetragenen Formaldehyds definierter Produktionskapazität:

- Massenstrom $m_1$ an insgesamt eingesetztem Anilin im Ausgangszustand: $m_1(A) \neq 0$ [z. B. in t/h],
- Massenstrom $m_2$ an insgesamt eingesetztem Formaldehyd im Ausgangszustand: $m_2(A) \neq 0$ [z. B. in t/h],
- Massenstrom $m_7$ an insgesamt eingesetztem sauren Katalysator im Ausgangszustand: $m_7(A) \neq 0$ [z. B. in t/h],
- Molares Verhältnis n(1)/n(2) von insgesamt eingesetztem Anilin (1) zu insgesamt eingesetztem Formaldehyd (2) im Ausgangszustand: n(1)/n(2)*(A)*,
- Molares Verhältnis n(7)/n(1) von insgesamt eingesetztem saurem Katalysator zu insgesamt eingesetztem Anilin (oft auch als *Protonierungsgrad = n(7)/n(1) · 100* % ausgedrückt) im Ausgangszustand: n(7)/n(1)*(A)*, sowie
- auf die Gesamtmasse an Di- und Polyaminen der Diphenylmethanreihe bezogener Massenanteil $\omega_{MMDA}$ an Diaminen der Diphenylmethanreihe im Ausgangszustand: $\omega_{MMDA}(A)$.

**Endzustand *E*** der Produktionsanlage mit angestrebter, durch die Menge des eingetragenen Formaldehyds definierter Produktionskapazität (wobei im Vergleich zum Ausgangszustand weniger oder mehr Formaldehyd eingetragen wird):

- Massenstrom an insgesamt eingesetztem Anilin im Endzustand: $m_1(E) \neq 0$ [z. B. in t/h],
- Massenstrom an insgesamt eingesetztem Formaldehyd im Endzustand: $m_2(E) \neq 0$ [z. B. in t/h],
- Massenstrom an insgesamt eingesetztem sauren Katalysator im Endzustand: $m_7(E) \neq 0$ [z. B. in t/h],
- Molares Verhältnis von insgesamt eingesetztem Anilin (1) zu insgesamt eingesetztem Formaldehyd (2) im Endzustand: n(1)/n(2)*(E)*,
- Molares Verhältnis von insgesamt eingesetztem saurem Katalysator zu insgesamt eingesetztem Anilin im Endzustand: n(7)/n(1)*(E),* sowie
- auf die Gesamtmasse an Di- und Polyaminen der Diphenylmethanreihe bezogener Massenanteil $\omega_{MMDA}$ an Diaminen der Diphenylmethanreihe, der für den Endzustand angestrebt ist: $\omega_{MMDA}(E)$.

**Übergangszustand *Ü*** zwischen Ausgangs- und Endzustand (entsprechend dem Zeitraum des Lastwechsels):

- Massenstrom an insgesamt eingesetztem Anilin im Übergangszustand: $m_1(Ü) \neq 0$ [z. B. in t/h],
- Massenstrom an insgesamt eingesetztem Formaldehyd im Übergangszustand: $m_2(Ü) \neq 0$ [z. B. in t/h],
- Massenstrom an insgesamt eingesetztem sauren Katalysator im Übergangszustand: $m_7(Ü) \neq 0$ [z. B. in t/h],
- Molares Verhältnis n(1)/n(2) von insgesamt eingesetztem Anilin (1) zu insgesamt eingesetztem Formaldehyd (2) im Übergangszustand: n(1)/n(2)*(Ü)*,
- Molares Verhältnis n(7)/n(1) von insgesamt eingesetztem saurem Katalysator (7) zu insgesamt eingesetztem Anilin im Übergangszustand: n(7)/n(1)*(Ü),*
- auf die Gesamtmasse an Di- und Polyaminen der Diphenylmethanreihe bezogener Massenanteil $\omega_{MMDA}$ an Diaminen der Diphenylmethanreihe im Übergangszustand: $\omega_{MMDA}(Ü)$.

Erfindungsgemäß beginnt der *Übergangszustand,* sobald $m_2$ ausgehend von $m_2(A)$ erhöht oder erniedrigt wird (dieser Zeitpunkt wird im Folgenden mit $t_1$ bezeichnet), und er endet, sobald $m_2$ den für $m_2(E)$ angestrebten Wert erreicht (dieser Zeitpunkt wird im Folgenden mit $t_2$ bezeichnet). Am Ende des so definierten Übergangszustandes (also bei $t = t_2$) ist der Betriebsparameter n(1)/n(2) so eingestellt, dass unter den Randbedingungen der veränderten Last die *Voraussetzungen* für die Produktion des Produkts MDA mit dem für den Endzustand angestrebten, geänderten Zweikerngehalt $\omega_{MMDA}(E)$ vorliegen, d. h. von nun an erfolgt die Produktion von *weiterem* MDA mit dem angestrebten Zweikerngehalt $\omega_{MMDA}(E)$. Bei $t = t_2$ gilt daher: **n(1)/n(2)*(Ü)* = n(1)/n(2)*(E)*.** Da jedoch in den Reaktoren noch Produktgemisch, das *vor $t_2$* produziert wurde, vorliegt (und teilweise, je nach Dauer des Übergangszustandes und der Verweilzeit in den Reaktoren, sogar noch Produkt, das *vor $t_1$* produziert wurde), entspricht die am Austritt des letzten Reaktors (3000-i) der Reaktorkaskade (3000) tatsächlich vorliegende Produktzusammensetzung **erst nach Verstreichen einer weiteren Zeitspanne $\Delta t$** (im Allgemeinen in der Größenordnung von Stunden bis wenigen Tagen) **dem für den Endzustand angestrebten Zweikerngehalt $\omega_{MMDA}(E)$.** Es gibt also nach Erreichen von $t_2$ einen Zeitraum, innerhalb dessen der im Austritt aus dem letzten Reaktor (3000-i) tatsächlich vorliegende Zweikerngehalt (der ein *Mittelwert* aus den wechselnden Produktzusammensetzungen während des Übergangszustandes ist) vom angestrebten Endwert $\omega_{MMDA}(E)$ abweicht. Spätestens jedoch, wenn alle Reaktoren der Reaktorkaskade (3000) mit MDA des angestrebten Zweikerngehalts $\omega_{MMDA}(E)$ durchgespült sind, entspricht der am Austritt des letzten Reaktors (3000-i) tatsächlich vorliegende Zweikerngehalt dem Wert von $\omega_{MMDA}(E)$. Dieser Zeitpunkt wird im Folgenden als $t_3$ bezeichnet. Bevorzugt beträgt die Zeitspanne $\Delta t = t_3 - t_2$ maximal 72 Stunden, besonders bevorzugt maximal 48 Stunden, ganz besonders bevorzugt maximal 24 Stunden.

Aufgrund des im erfindungsgemäßen Verfahren eingesetzten Mindestwerts für n(1)/n(2) von 1,6, wird die Ausbeute an Zielprodukt MDA durch den eingebrachten Mengenstrom an insgesamt eingesetztem *Formaldehyd (2)* limitiert. Der zur Herstellung eines bestimmten Produkttyps (d. h. eines durch eine bestimmte Isomerenzusammensetzung des MMDA und ein bestimmtes Verhältnis von MMDA zu PMDA charakterisiertes Gemisch aus Di- und Polyaminen der

Diphenylmethanreihe) unter den gegebenen Randbedingungen der Produktionsanlage (10 000) *maximal möglichen Mengenstrom an insgesamt eingesetztem Formaldehyd (2):*

- $m_2(N)$ [z. B. in t/h],
  wird hier und im Folgenden als **Nennlast** bezeichnet.

Der maximal mögliche Mengenstrom an insgesamt eingesetztem Formaldehyd (2) kann je nach herzustellendem MDA-Produkttyp schwanken. Sollte die Nennlast im Endzustand von der im Ausgangszustand signifikant abweichen, so ist die Nennlast *im Ausgangszustand,* $m_2(N, A)$ maßgeblich für die Zwecke der vorliegenden Erfindung (d. h. insbesondere für den Zweck der Quantifizierung der Laständerung; siehe unten für Details). Der Wert für $m_2(N, A)$ ist bei einer gegebenen Produktionsanlage entweder von vorne herein bekannt (etwa weil die Anlage für die Produktion einer bestimmten Menge eines bestimmten Produkttyps pro Stunde ausgelegt wurde) oder kann vom Fachmann aus den bekannten Randbedingungen der Anlage (Anzahl und Größe der vorhandenen Apparate und dgl.) für die bekannten Betriebsparameter des maßgeblichen Ausgangszustands (n(1)/n(2)*(A)*, n(7)/n(1)*(A)*,) leicht bestimmt werden. In der Praxis wird statt $m_2(N)$ häufig der entsprechende *Produkt*mengenstrom *an MDA* (z. B. in t/h) angegeben, der unter der Annahme der Auslegungsparameter für die Produktionsanlage (u. A. n(1)/n(2)-Verhältnis, n(7)/n(1)-Verhältnis etc.) zu erwarten ist. Statt **Nennlast** spricht man daher auch häufig von **Nenn-Produktionskapazität** oder auch kurz **Nennkapazität.** Die Begriffe "Last" und "(Produktions-)Kapazität" meinen in diesem Zusammenhang letztlich das Gleiche, haben jedoch unterschiedliche Bezugspunkte (Mengenstrom an *Ausgangsmaterial* in einem und Mengenstrom an *aus diesem Ausgangsmaterial zu erwartendem Zielprodukt* im anderen Fall).

Die **tatsächlich vorliegende Last** (oder - siehe die vorigen Erläuterungen - die tatsächlich vorliegende **Produktionskapazität** oder kurz **Kapazität)** im Ausgangs- bzw. Endzustand, definiert durch den im Ausgangs- bzw. Endzustand tatsächlich vorliegenden Massenstrom an insgesamt eingesetztem Formaldehyd, lässt sich dann *in Relation zur Nennlast* wie folgt ausdrücken:

- $$m_2(A) = X(A) \cdot m_2(N) \text{ [z. B. in t/h];}$$

- $$m_2(E) = X(E) \cdot m_2(N) \text{ [z. B. in t/h].}$$

In beiden Fällen ist X ein dimensionsloser Multiplikator größer als 0 und kleiner als oder gleich 1, der die tatsächliche Last zur Nennlast in Beziehung setzt. Bei einem Wechsel von Produktion mit halber Nennlast ("Halblast") auf Produktion mit Nennlast ("Volllast") gilt demnach $X(A) = ½$ und $X(E) = 1$. Im Falle von Laststeigerungen gilt $X(E) > X(A)$, im Falle von Lastreduzierungen gilt $X(E) < X(A)$.

Ein quantitatives Maß für die **Größe einer Laständerung** ist der **Betrag der Differenz von X(*E*) und X(*A*):**

- $$|X(E) - X(A)| \; (= |X(A) - X(E)|) = \mathbf{\Delta X}$$

Von einem **Lastwechsel** wird **im Sinne dieser Erfindung** gesprochen bei einem Wert für $\Delta$**X** von größer oder gleich 0,10, bevorzugt von größer oder gleich 0,15, besonders bevorzugt von größer oder gleich 0,20, ganz besonders bevorzugt von größer oder gleich 0,25. Das erfindungsgemäße Verfahren umfasst mindestens einen so definierten Lastwechsel.

Das erfindungsgemäße Verfahren umfasst auch mindestens eine Änderung des Zweikerngehalts. Von einer **Änderung des Zweikerngehalts im Sinne dieser Erfindung** wird dann gesprochen, wenn für den angestrebten Endwert des Zweikemgehalts, $\omega_{MMDA}(E)$, gilt:
$\omega_{MMDA}(E) \geq 1{,}05 \cdot \omega_{MMDA}(A)$, bevorzugt $\omega_{MMDA}(E) \geq 1{,}10 \cdot \omega_{MMDA}(A)$, besonders bevorzugt $\omega_{MMDA}(E) \geq 1{,}15 \cdot \omega_{MMDA}(A)$ (Erhöhung des Zweikerngehalts) bzw.

$\omega_{MMDA}(E) \leq 0{,}95 \cdot \omega_{MMDA}(A)$, bevorzugt $\omega_{MMDA}(E) \leq 0{,}90 \cdot \omega_{MMDA}(A)$, besonders bevorzugt $\omega_{MMDA}(E) \leq 0{,}85 \cdot \omega_{MMDA}(A)$ (Senkung des Zweikerngehalts).

Der Zweikerngehalt $\omega_{MMDA}$ ist dabei stets der Zweikerngehalt des aus dem letzten Reaktor (3000-i) der Reaktorkaskade (3000) austretenden MDA.

In allen oben genannten Betriebszuständen wird n(1)/n(2) auf einen Wert größer oder gleich 1,6 eingestellt. Bezüglich des Ausdrucks *"insgesamt eingesetzter saurer Katalysator"* gilt das oben für Anilin und Formaldehyd Gesagte entsprechend: Gemeint ist die Gesamtmenge an saurem Katalysator. Diese kann (und wird bevorzugt) vollständig dem Reaktor 3000-1 zugeführt werden; es ist aber auch möglich, diesem Reaktor nur den Hauptteil des insgesamt eingesetzten sauren Katalysators zuzuführen und kleinere Anteile zusätzlich in die in Strömungsrichtung folgenden Reaktoren

zuzudosieren.

Erfindungsgemäß wird die Temperatur im in Strömungsrichtung ersten Reaktor (3000-1) stets so gewählt, dass diese im Bereich von 25,0 °C bis 65,0 °C, bevorzugt 30,0 °C bis 60,0 °C, liegt. Dies gilt in allen Betriebszuständen, also im Ausgangs-, Übergangs- und Endzustand.

Erfindungsgemäß wird ferner die Temperatur in den in Strömungsrichtung nachfolgenden Reaktoren (3000-2, 3000-3, ... 3000-i) jeweils auf einen Wert eingestellt, der um mehr als 2,0 °C oberhalb von $T_{3000-1}$ liegt. Auch dies gilt in allen Betriebszuständen, also im Ausgangs-, Übergangs- und Endzustand.

Das molare Verhältnis von insgesamt eingesetztem saurem Katalysator zu insgesamt eingesetzten Anilin, n(7)/n(1), weicht erfindungsgemäß im Endzustand von dem im Ausgangszustand um maximal 10 %, bevorzugt um maximal 5 % ab, d. h. es gilt:

$0{,}90 \cdot n(7)/n(1)(A) \leq n(7)/n(1)(E) \leq 1{,}10 \cdot n(7)/n(1)(A)$, bevorzugt
$0{,}95 \cdot n(7)/n(1)(A) \leq n(7)/n(1)(E) \leq 1{,}05 \cdot n(7)/n(1)(A)$.

*Während* des Übergangszustandes *Ü* (also im Zeitraum von $t_1$ bis $t_2$) können größere Abweichungen vom Ausgangszustand zugelassen werden. Hier gilt:

$0{,}85 \cdot n(7)/n(1)(A) \leq n(7)/n(1)(\ddot{U}) \leq 1{,}15 \cdot n(7)/n(1)(A)$, bevorzugt
$0{,}90 \cdot n(7)/n(1)(A) \leq n(7)/n(1)(\ddot{U}) \leq 1{,}10 \cdot n(7)/n(1)(A)$;

Spätestens am Ende des Übergangszustandes, also zu dem Zeitpunkt, an dem $m_2$ den für $m_2(E)$ angestrebten Wert erreicht hat (Zeitpunkt $t_2$), ist die Abweichung jedoch, wie zuvor geschildert, auf maximal 10 %, bevorzugt maximal 5 %, zu begrenzen. Besonders bevorzugt wird das Verhältnis n(7)/n(1) im Endzustand auf den gleichen Wert eingestellt wie im Ausgangszustand (d.h. $n(7)/n(1)(E) = n(7)/n(1)(A)$), und lediglich im Übergangszustand wird gegebenenfalls ein vom Ausgangszustand in den zuvor definierten Grenzen abweichender Wert zugelassen.

Ferner wird während des Übergangszustandes *Ü*:

(i) die Temperatur im in Strömungsrichtung ersten Reaktor (3000-1) aus Schritt (A-II) auf einen Wert eingestellt, der von der Temperatur in diesem Reaktor während des Ausgangszustands *A* um maximal 10,0 °C, bevorzugt um maximal 5,0 °C, besonders bevorzugt um maximal 2,0 °C abweicht, wobei die besonders bevorzugte maximale Abweichung um ± 2,0 °C für alle praktischen Zwecke als Gleichhalten der Temperatur anzusehen ist (im Rahmen der vorliegenden Erfindung gelten geringfügige nominelle Abweichungen von einer Temperatur im Bereich von bis zu ± 2,0 °C als ein - im Wesentlichen - *Gleichhalten der Temperatur*; Änderungen von größer als 2,0 °C gelten dagegen als *signifikante Temperaturänderungen*);

(ii-1) **im Fall von $m_2(E) > m_2(A)$,** die Temperatur in mindestens einem der in Strömungsrichtung nachfolgenden Reaktoren (3000-2, ..., 3000-i) im Vergleich zum Ausgangszustand *A* um mehr als 2,0 °C erhöht wird, sodass die angestrebte Endtemperatur spätestens zu dem Zeitpunkt, an dem $m_2$ den für $m_2(E)$ angestrebten Wert erreicht hat (also bei $t = t_2$), vorliegt, und in allen Reaktoren (3000-2, ..., 3000-i), in denen die Temperatur nicht erhöht wird, im Bereich einer Schwankungsbreite von ± 2,0 °C gleich gehalten;

(ii-2) **im Fall von $m_2(E) < m_2(A)$,** die Temperatur in mindestens einem der in Strömungsrichtung nachfolgenden Reaktoren (3000-2, ..., 3000-i) im Vergleich zum Ausgangszustand *A* um mehr als 2,0 °C erniedrigt wird, sodass die angestrebte Endtemperatur spätestens zu dem Zeitpunkt, an dem $m_2$ den für $m_2(E)$ angestrebten Wert erreicht hat (also bei $t = t_2$), vorliegt, und in allen Reaktoren (3000-2, ..., 3000-i), in denen die Temperatur nicht erniedrigt wird, im Bereich einer Schwankungsbreite von ± 2,0 °C gleich gehalten;

(iii-1) **im Fall von $\omega_{MMDA}(E) \geq 1{,}05 \cdot \omega_{MMDA}(A)$,** $n(1)/n(2)(\ddot{U})$ so eingestellt wird, dass zu dem Zeitpunkt, an dem $m_2$ den für $m_2(E)$ angestrebten Wert erreicht hat (also bei $t = t_2$), gilt:

$1{,}05 \cdot n(1)/n(2)(A) \leq n(1)/n(2)(\ddot{U}) \leq 2{,}00 \cdot n(1)/n(2)(A)$, bevorzugt
$1{,}10 \cdot n(1)/n(2)(A) \leq n(1)/n(2)(\ddot{U}) \leq 1{,}60 \cdot n(1)/n(2)(A)$;

wobei während des gesamten Übergangszustandes vor Erreichen des Zeitpunkts $t_2$ stets gilt:

$0{,}80 \cdot n(1)/n(2)(A) \leq n(1)/n(2)(\ddot{U}) \leq 2{,}50 \cdot n(1)/n(2)(A)$, bevorzugt
$0{,}90 \cdot n(1)/n(2)(A) \leq n(1)/n(2)(\ddot{U}) \leq 2{,}00 \cdot n(1)/n(2)(A)$;

(iii-2) **im Fall von $\omega_{MMDA}$*(E)* ≤ 0,95 · $\omega_{MMDA}$*(A)*,** n(1)/n(2)*(Ü)* so eingestellt wird, dass zu dem Zeitpunkt, an dem $m_2$ den für $m_2$*(E)* angestrebten Wert erreicht hat (also bei t = $t_2$), gilt:

0,50 · n(1)/n(2)*(A)* ≤ n(1)/n(2)*(Ü)* ≤ 0,95 · n(1)/n(2)*(A)*, bevorzugt
0,60 · n(1)/n(2)*(A)* ≤ n(1)/n(2)*(Ü)* ≤ 0,90 · n(1)/n(2)*(A)*

wobei während des gesamten Übergangszustandes vor Erreichen des Zeitpunkts $t_2$ stets gilt:

0,40 · n(1)/n(2)*(A)* ≤ n(1)/n(2)*(Ü)* ≤ 2,50 · n(1)/n(2)*(A)*, bevorzugt
0,50 · n(1)/n(2)*(A)* ≤ n(1)/n(2)*(Ü)* ≤ 2,00 · n(1)/n(2)*(A)*.

Erfindungsgemäß ist für den Reaktor 3000-1 *während des Übergangszustands* eine signifikante Abweichung von der Temperatur im Ausgangszustand möglich. In diesem Reaktor machen sich Änderungen der freigesetzten Wärmemenge als Folge der Änderung der Last verstärkt bemerkbar. (Wesentliche Beiträge zur Exothermie sind zum einen die Neutralisationswärme, die bei der Reaktion der Säure mit den aminischen Komponenten des Reaktionsgemisches frei wird, sowie die einsetzende säurekatalysierte Umlagerung des Aminals.) Dem kann erforderlichenfalls durch größere Toleranzen bei der Temperatur im Übergangszustand Rechnung getragen werden. Es ist jedoch bevorzugt, zum Ende des Übergangszustandes (also wenn $m_2$ den für $m_2$*(E)* angestrebten Wert erreicht hat; Zeitpunkt $t_2$), die Temperatur im Reaktor 3000-1 wieder auf einen Wert einzustellen, der innerhalb einer Schwankungsbreite von ± 2,0 °C derjenigen des Ausgangszustandes entspricht.

Die *Temperaturerhöhung* in mindestens einem der Reaktoren 3000-2, ..., 3000-*i im Falle einer Laststeigerung* sollte möglichst rasch vorgenommen werden, um die Verkürzung der Verweilzeit bestmöglich ausgleichen zu können. Bevorzugt wird die für den Endzustand angestrebte Temperatur bereits bei $t_1$ als neue Soll-Temperatur eingestellt, sodass die tatsächlich vorliegende Ist-Temperatur in kürzest möglicher Zeit, spätestens jedoch bei $t_2$, den für den Endzustand angestrebten Wert annimmt.

Die *Temperaturerniedrigung* in mindestens einem der Reaktoren 3000-2, ..., 3000-i *im Falle einer Lastsenkung* sollte dagegen möglichst langsam vorgenommen werden, um die Bildung nicht umgelagerter Produkte zu vermeiden. Bevorzugt wird die für den Endzustand angestrebte Temperatur erst bei $t_2$ erreicht.

Erfindungsgemäß wesentlich ist, dass am Ende des Übergangszustandes (also wenn die für den Endzustand angestrebte Last eingestellt ist) im Falle einer Steigerung des Zweikerngehalts das molare Verhältnis von insgesamt eingesetztem Anilin zu insgesamt eingesetztem Formaldehyd **(n(1)/n(2))** gegenüber dem Ausgangszustand *erhöht* ist. Im Fall einer Senkung des Zweikerngehalts wird umgekehrt verfahren (Erniedrigung von n(1)/n(2)). Grundsätzlich kann eine Änderung des Zweikerngehalts sowohl mit einer Laststeigerung als auch mit einer Lastsenkung verbunden werden. Abhängig von der Ausgangsituation kann es jedoch bevorzugte Kombinationen geben:
Insbesondere dann, wenn die Last im Ausgangszustand, $m_2$*(A)*, bereits auf einem Wert ist, der der Nennlast nahekommt oder sogar gleich der Nennlast ist, ist es bevorzugt - unter Umständen wie eingangs beschrieben sogar erforderlich -, eine angestrebte *Erhöhung des Zweikerngehalts* (also im Fall von $\omega_{MMDA}$*(E)* ≥ 1,05 · $\omega_{MMDA}$*(A)*) mit einer *Lasterniedrigung* zu verbinden.

Auch der umgekehrte Fall, die Verbindung einer *Senkung des Zweikerngehalts* (also im Fall von $\omega_{MMDA}$*(E)* ≤ 0,95 · $\omega_{MMDA}$*(A)*) mit einer *Laststeigerung,* kann bevorzugt sein, nämlich dann, wenn durch die mit der Verringerung des Zweikerngehalts einhergehende Erniedrigung des Verhältnisses n(1)/n(2) mehr Spielraum für eine Erhöhung der Last zur Verfügung steht (und dies mit einer entsprechenden Nachfrage nach dem Zielprodukt einhergeht). Selbstverständlich ist die Auswahl der Kombination von Laständerung und Zweikerngehaltänderung stark vom Ausgangszustand abhängig: Beträgt die Last $m_2$ im Ausgangszustand beispielsweise bereits nur 50 % der Nennlast (sog. "Halblast"), wird man im Allgemeinen unabhängig von der angestrebten Änderung des Zweikerngehalts bestrebt sein, die Last zu steigern (es sei denn, die Nachfrage nach dem Produkt mit geänderter Zusammensetzung ist sehr gering).

Die erfindungsgemäße Vorgehensweise ist in **FIG. 1a-c** exemplarisch am Beispiel einer *Senkung des Zweikerngehalts in Verbindung mit einer Laststeigerung* illustriert. Diese und die übrigen Zeichnungen sollen lediglich das Grundprinzip der Erfindung verdeutlichen und erheben keinen Anspruch auf Maßstabstreue. In FIG. la-c ist der Zweikerngehalt $\omega_{MMDA}$ als Funktion der Zeit t aufgetragen. Im Ausgangszustand *A* mit gegebenen Werten für die Betriebsparameter n(1)/n(2) = n(1)/n(2)*(A)* und $m_2$ = $m_2$*(A)* (sowie - nicht gezeigt - n(7)/n(1) = n(7)/n(1)*(A)*) entspricht $\omega_{MMDA}$ dem Ausgangswert $\omega_{MMDA}$*(A)*. Durch die im folgenden Absatz noch näher erläuterten erfindungsgemäßen Änderungen der Betriebsparameter im Übergangszustand (beginnend bei t = $t_1$ und endend bei t = $t_2$) sinkt $\omega_{MMDA}$ im Laufe der Zeit auf den Endwert $\omega_{MMDA}$*(E)*. Die Endwerte für die Betriebsparameter n(1)/n(2) und $m_2$ sind bei $t_2$ erreicht. Bei $t_2$ liegen also die *Voraussetzungen* für die Produktion von weiterem MDA mit dem angestrebten Zweikerngehalt $\omega_{MMDA}$*(E)* vor. Wie oben im Zusammenhang mit der Definition des Übergangszustandes erläutert, erreicht die Zusammensetzung des im Austritt aus dem letzten Reaktor (3000-i) der Reaktorkaskade (3000) tatsächlich vorliegenden MDA jedoch erst einige Zeit nach $t_2$, nämlich bei $t_3$, diesen angestrebten Endwert $\omega_{MMDA}$*(E)*. Letzteres gilt selbstverständlich nicht nur für die

in den FIG. la-c gezeigten beispielhaften Fälle, sondern *für alle denkbaren Kombinationen von Lastwechsel und Änderung des Zweikerngehalts,* also beispielsweise auch bei einer angestrebten *Steigerung* von $\omega_{MMDA}$.

Im Anfangszustand beträgt die Last $m_2 = m_2(A)$. Zum Zeitpunkt $t = t_1$ *(= Beginn des Übergangszustandes)* wird die Last erhöht, bis diese zum Zeitpunkt $t = t_2$ (= Ende des Übergangszustandes) den für den Endzustand angestrebten Wert $m_2 = m_2(E)$ erreicht, d. h. es gilt - und zwar für alle denkbaren Ausgestaltungen der Erfindung und nicht nur in den in FIG. la-c exemplarisch dargestellten Fällen - $\mathbf{m_2(t=t_2) = m_2(E)}$ **und** $\mathbf{n(1)/n(2)(\ddot{U})(t=t_2) = n(1)/n(2)(E)}$. Die Absenkung von n(1)/n(2) kann, wie in **FIG. 1a** gezeigt, kontinuierlich erfolgen. Dies ist jedoch nicht zwingend; es ist beispielsweise auch möglich, wie in **FIG. 1b** gezeigt, n(1)/n(2) zunächst zu erhöhen (indem $m_1$ zunächst in stärkerem Ausmaß erhöht wird als $m_2$) und erst zeitversetzt auf den angestrebten Endwert zu senken, was zu einer zwischenzeitlichen Steigerung von n(1)/n(2) führt. Wesentlich ist lediglich, dass der für das Verhältnis n(1)/n(2) angestrebte Endwert während des Übergangszustandes so eingestellt wird, dass dieser angestrebte Endwert n(1)/n(2) = n(1)/n(2)*(E)* zum Zeitpunkt $t = t_2$ (also zu dem Zeitpunkt, an dem $m_2$ den für $m_2(E)$ angestrebten Wert erreicht hat) vorliegt. Dies ist nicht auf den in FIG. la-c exemplarisch dargestellten Fall der *Senkung des Zweikerngehalts in Verbindung mit einer Laststeigerung* beschränkt, sondern gilt *für alle denkbaren Kombinationen von Lastwechsel und Änderung des Zweikerngehalts.* Die Einstellung des angestrebten Endwerts n(1)/n(2)*(E)* kann dabei stufenweise oder, wie in FIG. la-c gezeigt, kontinuierlich erfolgen, wobei die kontinuierliche Einstellung bevorzugt ist. Die jeweils erforderlichen Anpassungen des Betriebsparameters $m_2$ und erforderlichenfalls $m_1$ und/oder $m_7$ werden bevorzugt zeitgleich begonnen und besonders bevorzugt so durchgeführt, dass die für das Ende des Lastwechsels (also der Zeitpunkt, an dem $m_2$ den für $m_2(E)$ angestrebten Wert erreicht hat) jeweils angestrebten Werte durch kontinuierliche Anpassung des jeweiligen Betriebsparameters eingestellt werden.

Eine leichte Zeitversetzung (im Allgemeinen in der Größenordnung von Sekunden, bevorzugt maximal 15 Sekunden) bei den erforderlichen Anpassungen von $m_1$ und $m_2$ ist jedoch möglich. Dies erfolgt insbesondere derart, dass im Falle einer Laststeigerung zunächst mit der Anpassung von $m_1$ begonnen wird (insofern eine solche Anpassung, im Allgemeinen Erhöhung, erforderlich ist) und dann erst mit geringer Zeitversetzung $m_2$ erhöht wird, und zwar derart, dass die erfindungsgemäßen Anforderungen an das Verhältnis n(1)/n(2) eingehalten werden. Im Falle einer Lastsenkung wird umgekehrt verfahren (Beginn mit der Anpassung (d. h. in diesem Fall Erniedrigung) von $m_2$, dann gegebenenfalls Anpassung, im Allgemeinen Erniedrigung, von $m_1$, selbstverständlich wieder unter der Maßgabe, dass die erfindungsgemäßen Anforderungen an das Verhältnis n(1)/n(2) eingehalten werden). Wird in dieser Ausführungsform bei einer Laststeigerung mit einer Erhöhung von $m_1$ begonnen, bevor mit der Erhöhung von $m_2$ begonnen wird, so steigt das n(1)/n(2)-Verhältnis bereits kurz vor $t = t_1$ (d. h. kurz vor Beginn des Übergangszustands wie erfindungsgemäß definiert) an. Allgemein gilt, dass wenn mit einer Anpassung von $m_1$ begonnen wird, *bevor* mit der gewünschten Änderung (Laststeigerung oder Lastsenkung) von $m_2$ begonnen wird (ein solcher Zeitpunkt ist in **FIG. 1c** gezeigt und dort mit $t_0$ gekennzeichnet), die Verhältnisse n(1)/n(2)*(A)* und n(7)/n(1)*(A)* für die Zwecke der vorliegenden Erfindung auf Basis des Wertes von $m_1$ *vor* Beginn der Anpassung von $m_1$ berechnet werden (vgl. FIG. 1c).

Nachfolgend werden Ausführungsformen der Erfindung näher beschrieben. Diese Ausführungsformen gelten, sofern im Einzelfall nicht anders vermerkt, für alle Verfahrensführungen der Erfindung. Dabei können verschiedene Ausführungsformen beliebig miteinander kombiniert werden, sofern sich für den Fachmann aus dem Zusammenhang nicht das Gegenteil ergibt.

**FIG. 2** zeigt eine für die Durchführung des erfindungsgemäßen Verfahrens geeignete Produktionsanlage (10 000).

**FIG. 3** zeigt einen Detailausschnitt der Reaktorkaskade (3000). Die lediglich optionale Zugabe weiteren sauren Katalysators (7) zu den dem Reaktor (3000-1) nachgeschalteten Reaktoren (3000-2) bis (3000-i) ist durch gestrichelte Pfeile dargestellt.

**Schritt (A-I)** des erfindungsgemäßen Verfahrens kann, sofern die übrigen erfindungsgemäßen Erfordernisse eingehalten werden, so durchgeführt werden wie aus dem Stand der Technik grundsätzlich bekannt. Dabei werden bevorzugt Anilin und wässrige Formaldehydlösung bei Molverhältnissen im Bereich von 1,6 bis 20, bevorzugt 1,6 bis 10 und besonders bevorzugt 1,6 bis 6,0, ganz besonders bevorzugt von 1,7 bis 5,5 und außerordentlich ganz besonders bevorzugt von 1,8 bis 5,0 bei Temperaturen von 20,0 °C bis 120,0 °C, bevorzugt 40,0 °C bis 110,0 °C und besonders bevorzugt 60,0 °C bis 100,0 °C zu Aminal und Wasser kondensiert. Der Reaktor des Schritts (A-I) wird bei Normaldruck oder im Überdruck betrieben. Bevorzugt liegt ein Druck von 1,05 bar bis 5,00 bar (absolut), ganz bevorzugt von 1,10 bar bis 3,00 bar und ganz besonders bevorzugt von 1,20 bar bis 2,00 bar (absolut) vor. Die Druckhaltung erfolgt durch Druckregelungsventile, oder indem man die Abgassysteme von Aminalreaktor (1000) und dem Überlauf des zur Phasentrennung nach erfolgter Umsetzung eingesetzten Aminalabscheiders (2000) verbindet. Der Aminalabscheider und der Ablauf der wässrigen Phase sind vorzugsweise beheizt, um Anbackungen zu verhindern.

Geeignete Anilinqualitäten sind z. B. beschrieben in EP 1 257 522 B1, EP 2 103 595 A1 und EP 1 813 598 B1. Es werden bevorzugt technische Qualitäten an Formalin (wässrige Lösung von Formaldehyd) mit 30,0 Massen-% bis 50,0 Massen-% Formaldehyd in Wasser eingesetzt. Jedoch sind auch Formaldehydlösungen mit niedrigeren oder höheren Konzentrationen oder auch der Einsatz gasförmigen Formaldehyds denkbar.

Die Phasentrennung von organischer Aminalphase und wässriger Phase erfolgt bevorzugt bei Temperaturen von 20,0 °C bis 120,0 °C, besonders bevorzugt von 40,0 °C bis 110,0 °C, und ganz besonders bevorzugt von 60,0 °C bis 100,0 °C, jeweils bevorzugt bei Umgebungsdruck oder bei einem gegenüber Umgebungsdruck geringfügig erhöhtem Druck (um bis zu 0,10 bar erhöht) durchgeführt werden.

**Schritt (A-II)** des erfindungsgemäßen Verfahrens kann, sofern die übrigen erfindungsgemäßen Erfordernisse eingehalten werden, so durchgeführt werden wie aus dem Stand der Technik grundsätzlich bekannt. Die Umlagerung des Aminals erfolgt in Gegenwart eines sauren Katalysators, üblicherweise einer starken Mineralsäure wie Salzsäure. Bevorzugt ist der Einsatz von Mineralsäure in einem molaren Verhältnis Mineralsäure zu Anilin von 0,0010 bis 0,90, bevorzugt 0,050 bis 0,50. Es können natürlich auch feste saure Katalysatoren, wie in der Literatur beschrieben, verwendet werden. Dabei kann Formaldehyd in eine Mischung von Anilin und saurem Katalysator gegeben und die Reaktionslösung durch stufenweises Erhitzen ausreagiert werden. Alternativ können auch Anilin und Formaldehyd zunächst vorreagiert werden und anschließend mit oder ohne vorhergehender Wasserabtrennung mit dem sauren Katalysator oder einem Gemisch von weiterem Anilin und saurem Katalysator versetzt werden, wonach die Reaktionslösung durch stufenweises Erhitzen ausreagiert wird. Diese Reaktion kann kontinuierlich oder diskontinuierlich nach einem der zahlreichen in der Literatur beschriebenen Verfahren ausgeführt werden (z. B. in EP 1 616 890 A1 oder EP 127 0544 A1).

Es ist möglich, dem ersten Reaktor 3000-1 weiteres Anilin und/oder weiteren Formaldehyd zuzuführen. Es ist ebenfalls möglich, den nachfolgenden Reaktoren 3000-2, ..., 3000-i noch geringe Mengen an Anilin und/oder Formaldehyd zuzuführen. Dies können jeweils frische Einsatzstoffe sein oder Rückführströme aus anderen Reaktoren. Die Hauptmenge an insgesamt eingesetztem Anilin und an insgesamt eingesetztem Formaldehyd wird jedoch in den "Aminalreaktor" 1000 eingeführt. Unabhängig davon, wie Anilin (1) und Formaldehyd (2) ggf. auf verschiedene Reaktoren verteilt werden, wird das erfindungsgemäße Verfahren bevorzugt so betrieben, dass das molare Verhältnis von insgesamt eingesetztem Anilin (1) zu insgesamt eingesetztem Formaldehyd (2), n(1)/n(2), in allen Betriebszuständen (*A, Ü, E*) stets einen Wert von 1,6 bis 20, bevorzugt von 1,6 bis 10 und besonders bevorzugt von 1,6 bis 6,0, ganz besonders bevorzugt von 1,7 bis 5,5 und außerordentlich ganz besonders bevorzugt von 1,8 bis 5,0 aufweist.

Bevorzugt wird der insgesamt eingesetzte saure Katalysator (7) komplett dem Reaktor 3000-1 zugeführt. Es ist aber auch möglich, einen Teil des insgesamt eingesetzten sauren Katalysators (7) einem oder mehreren der in Strömungsrichtung folgenden Reaktoren 3000-2, ..., 3000-i zuzuführen.

Als saurer Katalysator (7) wird im erfindungsgemäßen Verfahren bevorzugt eine Mineralsäure, insbesondere Salzsäure eingesetzt. Geeignete Salzsäurequalitäten sind z. B. in EP 1 652 835 A1 beschrieben.

Als Reaktoren 3000-1, 3000-2, ... 3000-i in Schritt (A) eignen sich in beiden Verfahrensführungen dem Fachmann bekannte Vorrichtungen wie Rührkessel und Rohrreaktoren:
Bei Rührkesselreaktoren ist die Temperatur im Allgemeinen über den gesamten Reaktorinhalt gleich, sodass es für die Zwecke der vorliegenden Erfindung keine Rolle spielt, an welcher Stelle die Temperatur gemessen wird. Bestehen wider Erwarten signifikante Temperaturunterschiede über den Reaktorinhalt, so ist die am Austritt der Reaktionsmischung aus dem Reaktor gemessene Temperatur die für die Zwecke der vorliegenden Erfindung maßgebliche Temperatur.

Liegt zwischen Eintritt der Reaktionsmischung in den Reaktor und Austritt der Reaktionsmischung aus dem Reaktor ein signifikanter Temperaturgradient, wie dies bei Rohrreaktoren der Fall sein kann, so ist die am Austritt der Reaktionsmischung aus dem Reaktor gemessene Temperatur die für die Zwecke der vorliegenden Erfindung maßgebliche Temperatur.

Bevorzugt nimmt die Temperatur in den Reaktoren der Reaktorkaskade 3000 von Reaktor 3000-1 bis Reaktor 3000-i zu, d. h. im letzten Reaktor 3000-i ist die Temperatur bevorzugt höher als im ersten Reaktor 3000-1, wobei die Temperatur in zwei aufeinanderfolgenden Reaktoren zwischen 3000-1 und 3000-i auch gleich sein kann, jedoch Temperatur jedes Reaktors 3000-2, 3000-3, ..., 3000-i nicht geringer ist als die des vorangehenden Reaktors. Besonders bevorzugt nimmt jedoch die Temperatur in den Reaktoren der Reaktorkaskade 3000 von Reaktor 3000-1 bis Reaktor 3000-i sukzessive zu, d. h. die Temperatur jedes Reaktors 3000-2, 3000-3, ..., 3000-i ist höher als die des vorangehenden Reaktors.

Es ist ebenfalls bevorzugt,

- $T_{3000\text{-}1}$ stets auf einen Wert von 25,0 °C bis 65,0 °C, besonders bevorzugt 30,0 °C bis 60,0 °C *und*
- die Temperatur in allen in Strömungsrichtung nachfolgenden Reaktoren (3000-2, ..., 3000-i) stets jeweils auf einen Wert von 35,0 °C bis 200,0 °C, besonders bevorzugt von 50,0 °C bis 180,0 °C,
  einzustellen.

Hierdurch wird es möglich, im Reaktor 3000-1 einen praktikablen Kompromiss zwischen dem Ziel *möglichst geringer Nebenproduktbildung, insbesondere im Hinblick auf N-Methyl- und N-Formyl-MDA,* einerseits (wird durch *geringere* Temperatur begünstigt) und dem Ziel, *eine die praktikable Verarbeitbarkeit des Produkts gewährleistende Viskosität des Reaktionsgemisches sicherzustellen* andererseits (wird durch *höhere* Temperatur begünstigt) zu erzielen.

Alle vorgenannten Angaben zu bevorzugten Temperaturen gelten in allen Betriebszuständen (*A, Ü, E*).

Die am Ende des Übergangszustandes (also wenn $m_2$ den für den Endzustand angestrebten Wert $m_2$*(E)* erreicht hat) jeweils vorliegenden Werte für n(1)/n(2), n(7)/n(1) und für die Temperatur eines jeden Reaktors j der Reaktorkaskade $T_{3000\text{-}j}$ werden bevorzugt für die Dauer der Produktion mit dem Formaldehyd-Massenstrom $m_2$*(E)* beibehalten. Bei der Temperatur $T_{3000\text{-}j}$ gelten in der Terminologie der vorliegenden Erfindung wie bereits erwähnt geringfügige Schwankungen im Bereich von $\pm$ 2,0 °C noch als Gleichhalten der Temperatur.

**Schritt (B-I)** kann, sofern die übrigen erfindungsgemäßen Erfordernisse eingehalten werden, so durchgeführt werden wie aus dem Stand der Technik grundsätzlich bekannt. Das Reaktionsgemisch enthaltend die Di- und Polyamine der Diphenylmethanreihe wird ggf. unter Zusatz von Wasser und / oder Anilin neutralisiert. Nach dem Stand der Technik erfolgt die Neutralisation üblicherweise bei Temperaturen von beispielsweise 90,0 °C bis 120,0 °C ohne Zusatz weiterer Substanzen. Sie kann aber auch auf einem anderen Temperaturniveau erfolgen, um z. B. den Abbau von störenden Nebenprodukten zu beschleunigen. Als Basen sind beispielsweise geeignet die Hydroxide der Alkali- und Erdalkalielemente. Vorzugsweise kommt wässrige NaOH zur Anwendung. Die zur Neutralisation eingesetzte Base wird bevorzugt in Mengen von größer als 100 %, besonders bevorzugt 105 % bis 120 % der für die Neutralisation des eingesetzten sauren Katalysators stöchiometrisch benötigten Menge eingesetzt (siehe EP 1 652 835 A1).

**Schritt (B-II)** kann, sofern die übrigen erfindungsgemäßen Erfordernisse eingehalten werden, so durchgeführt werden wie aus dem Stand der Technik grundsätzlich bekannt. Das neutralisierte Reaktionsgemisch enthaltend die Di- und Polyamine der Diphenylmethanreihe wird in eine organische Phase enthaltend Di- und Polyamine der Diphenylmethanreihe und eine wässrige Phase aufgetrennt. Dies kann durch die Zugabe von Anilin und / oder Wasser unterstützt werden. Wird die Phasentrennung durch Zugabe von Anilin und / oder Wasser unterstützt, so erfolgt deren Zugabe bevorzugt bereits unter intensivem Mischen in der Neutralisation. Dabei kann die Vermischung in Mischstrecken mit statischen Mischern, in Rührkesseln oder Rührkesselkaskaden oder aber in einer Kombination von Mischstrecken und Rührkessel erfolgen. Das neutralisierte und durch Zugabe von Anilin und / oder Wasser verdünnte Reaktionsgemisch wird anschließend bevorzugt einem Apparat zugeführt, der aufgrund seiner Konfiguration und / oder Einbauten besonders zur Auftrennung in eine organische Phase enthaltend MDA und eine wässrige Phase geeignet ist, bevorzugt Phasentrenn- oder Extraktionsvorrichtungen entsprechend dem Stand der Technik, wie sie beispielsweise in Mass-Transfer Operations, 3rd Edition, 1980, McGraw-Hill Book Co, S. 477 bis 541, oder Ullmann's Encyclopedia of Industrial Chemistry (Vol. 21, Liquid-Liquid Extraction, E. Müller et al., Seite 272-274, 2012 Wiley-VCH Verlag GmbH & Co. KGaA, Weinheim, DOI: 10.1002/14356007.b03_06.pub2) oder in Kirk-Othmer Encyclopedia of Chemical Technology (siehe "http://onlinelibrary.wiley.com/book/10.1002/0471238961", Published Online: 15 Juni 2007, Seite 22-23) beschrieben sind (Mixer-Settler-Kaskade oder Absetzbehälter).

Wird in Schritt (A-II) während des Übergangszustandes der Mengenstrom an saurem Katalysator signifikant erhöht, so wird selbstverständlich auch der Mengenstrom an in Schritt (B-I) eingesetzter Base entsprechend erhöht (und zwar im gleichen Zeitrahmen), damit das erfindungsgemäße Erfordernis des stöchiometrischen Überschusses an Base stets, also auch im Übergangszustand, erfüllt ist. Wird in Schritt (A-II) während des Übergangszustandes der Mengenstrom an saurem Katalysator signifikant erniedrigt, so wird bevorzugt auch der Mengenstrom an in Schritt (B-I) eingesetzter Base entsprechend erniedrigt (und zwar ebenfalls im gleichen Zeitrahmen), um unnötige Salzfrachten zu vermeiden.

Bevorzugt schließen sich weitere Aufarbeitungsschritte an Schritt (B-II) an, nämlich:

> **Schritt (B-III):** Waschen der organischen Phase (11) in einer Wascheinrichtung (6000, "Waschbehälter") mit Waschflüssigkeit (13) gefolgt von
>
> **Schritt (B-IV):** Auftrennen des in Schritt (B-III) erhaltenen Gemisches (14) in einer Auftrenneinrichtung (7000, "Waschwasserabscheider") in eine organische Phase (16) umfassend Di- und Polyamine der Diphenylmethanreihe und eine wässrige Phase (15);
>
> **Schritt (B-V):** Destillieren der organischen Phase (16) aus Schritt (B-IV) in einer Destillationsvorrichtung (8000) unter Erhalt der Di- und Polyamine der Diphenylmethanreihe (18), wobei ein Wasser und Anilin enthaltender Strom (17) abgetrennt wird.

Diese Schritte können so durchgeführt werden wie aus dem Stand der Technik grundsätzlich bekannt. Insbesondere ist es bevorzugt, auch diese Schritte kontinuierlich (wie zuvor definiert) durchzuführen.

Besonders bevorzugt ist es, dass sich eine Wäsche (B-III) der organischen Phase (11) mit Wasser (13) und eine neuerliche Abscheidung der Wasserphase (15) zur Entfernung von Restgehalten an Salz (bevorzugt wie in DE-A-2549890, Seite 3 beschrieben) anschließt. Nach Austritt aus der Phasentrennung in Schritt (B-IV) hat die organische Phase (16) enthaltend Di- und Polyamine der Diphenylmethanreihe üblicherweise eine Temperatur von 80,0 °C bis 150,0 °C.

Aus der so erhaltenen organischen Phase enthaltend Di- und Polyamine der Diphenylmethanreihe wird destillativ Wasser und Anilin abgetrennt, wie in EP 1 813 597 B1 beschrieben. Die organische Phase hat bevorzugt eine Zusammensetzung, bezogen auf die Masse des Gemisches, von 5,0 bis 15 Massen-% Wasser, und, je nach Einsatzverhältnissen von Anilin und Formaldehyd, 5,0 bis 90 Massen-%, bevorzugt 5,0 bis 40 Massen-%, Anilin und 5,0 bis 90 Massen-%, bevorzugt 50 bis 90 Massen-%, Di- und Polyamine der Diphenylmethanreihe.

Besonders bevorzugt umfasst das erfindungsgemäße Verfahren ferner einen **weiteren Schritt (C),** in welchem eine Rückführung des Wasser und Anilin enthaltenden Stroms (17), ggf. nach Aufarbeitung, in Schritt (A-I) und/oder, sofern die optionale Zugabe weiteren Anilins (1) in Schritt (A-II) durchgeführt wird, in Schritt (A-II), vorgenommen wird.

In allen Ausführungsformen der Erfindung ist die Einstellung der Betriebsparameter auf die für den Endzustand angestrebten Werte mit dem Ende des Lastwechsels (also sobald der Massenstrom an insgesamt eingesetztem Formaldehyd $m_2$ den für den Endzustand angestrebten Wert $m_2(E)$ erreicht hat; Zeitpunkt $t_2$), abgeschlossen. Die Werte von n(1)/n(2) und der Temperatur in jedem Reaktor der Reaktorkaskade (3000) werden daher stufenweise oder kontinuierlich, bevorzugt kontinuierlich, auf die für den Endzustand angestrebten Werte eingestellt. Das gleiche gilt für den Wert n(7)/n(1), sofern dieser Wert verändert wird. Bei *stufenweiser Einstellung* des jeweiligen Parameters umfasst die Einstellung bevorzugt mehrere Stufen, d. h. dass beispielsweise eine angestrebte Endtemperatur über einen oder mehrere Zwischenwerte, die jeweils für einen bestimmten Zeitraum beibehalten werden, zwischen der Ausgangs- und der angestrebten Endtemperatur eingestellt wird. Der Begriff *"kontinuierliche Einstellung"* umfasst auch den Fall, dass der jeweilige Parameter zunächst in "entgegengesetzter Richtung" variiert wird, wie in FIG. 1b-c gezeigt.

In allen Ausführungsformen der Erfindung ist es ferner bevorzugt, den Lastwechsel und die parallel durchgeführte Veränderung der für den Zweikerngehalt maßgeblichen Betriebsparameter zeitlich auf maximal 120 Minuten zu beschränken, d. h. der Zeitraum, innerhalb dessen der Massenstrom an insgesamt eingesetztem Formaldehyd $m_2$ ausgehend von $m_2(A)$ auf den für den Endzustand angestrebten Wert $m_2(E)$ eingestellt wird (= Zeitdauer des Übergangszustandes = Zeitraum von $t_1$ bis $t_2$), ist bevorzugt auf 120 Minuten zu begrenzen. Die Mindestdauer des Übergangszustandes beträgt dabei bevorzugt 1,00 Minuten, besonders bevorzugt 5,00 Minuten und ganz besonders bevorzugt 30,0 Minuten. Die avisierte Änderung der für den Zweikerngehalt maßgeblichen Betriebsparameter findet dabei im gleichen Zeitraum statt.

Detaillierte Ausgestaltungen der Erfindung werden in den beigefügten Beispielen im Detail erläutert.

Die erfindungsgemäß erhaltenen Di- und Polyamine der Diphenylmethanreihe können nach den bekannten Methoden unter inerten Bedingungen mit Phosgen in einem organischen Lösungsmittel zu den entsprechenden Di- und Polyisocyanaten der Diphenylmethanreihe, dem MDI, umgesetzt werden. Dabei kann die Phosgenierung nach einem der aus dem Stand der Technik bekannten Verfahren durchgeführt werden (z.B. DE-A-844896 oder DE-A-19817691).

Durch die erfindungsgemäße Vorgehensweise ergeben sich die folgenden Vorteile für die Herstellung von MDA:

i) Die Produktivität der MDA-Anlage ist höher, weil der Anfall von nichtspezifikationsgerechter Ware minimiert wird.
ii) Es entstehen weniger Nebenprodukte mit Acridin- und Acridan-Struktur und/oder N-Formyl- und N-Methyl-MDA. Höhere Anteile an diesen Nebenprodukten können im Folgeprodukt MDI zu Qualitätsproblemen führen.

Somit ermöglicht die erfindungsgemäße Verfahrensweise während eines nicht-stationären Zustandes (während des Übergangszustandes) einen technisch reibungslosen Lastwechsel in Verbindung mit einer Änderung des Zweikerngehalts ohne anschließende Ausfallzeiten im darauffolgenden stationären Zustand (dem Endzustand) mit gleichbleibend hoher Qualität des angestrebten MDA-Endprodukts. Das erfindungsgemäße Verfahren ermöglicht eine zügige Durchführung eines mit einer Änderung des Zweikerngehalts verbundenem Lastwechsels und somit ein schnelles Reagieren auf Ereignisse wie Rohstoffmangel, veränderte Anforderungen an die Produktzusammensetzung etc.

## Beispiele

Die in den Beispielen geschilderten Ergebnisse für den Zweikerngehalt, die Isomerenzusammensetzung sowie den Gehalt an N-Methyl-4,4'-MDA beruhen auf Berechnungen. Die Berechnungen basieren teils auf theoretischen Modellen und teils auf in realen Betriebsversuchen gesammelten Prozessdaten, durch deren statistische Auswertung eine mathematische Korrelation von Fahrparametern und Ergebnis (z. B. Zweikerngehalt) erstellt wurde. Der Gehalt an N-Formyl-4,4'-MDA wird auf Basis betrieblicher Erfahrungswerte angegeben. Alle angegebenen Prozent- und ppm-Werte sind Massenanteile bezogen auf die Gesamtmasse des jeweiligen Stoffstroms. Die Massenanteile in den realen Betriebsversuchen, welche die Basis für die theoretischen Modelle lieferten, wurden durch HPLC ermittelt.

Reaktortemperaturen beziehen sich auf die Temperatur des jeweiligen Verfahrensproduktes am Austritt aus dem Reaktor.

Das hergestellte MDA weist in allen Beispielen einen Rest-Anilingehalt im Bereich von 50 ppm bis 100 ppm und einen Wassergehalt im Bereich von 200 ppm bis 300 ppm auf.

## A. Lastreduzierung ausgehend von Produktion mit Nennlast

### I. Ausgangszustand: Beschreibung der für die Herstellung von MDA bei Nennlast gewählten Bedingungen

In einem kontinuierlichen Reaktionsprozess werden 23,20 t/h Einsatzanilin (enthaltend 90,0 Massen-% Anilin, 1) und 9,60 t/h 32%ige wässrige Formaldehydlösung (entsprechend einem molaren Verhältnis von Anilin (1): Formaldehyd (2) von 2,25 : 1 vermischt) und bei einer Temperatur von 90,0 °C und einem Druck von 1,40 bar (absolut) in einem gerührten Reaktionskessel (1000) zum Aminal (3) umgesetzt. Der Reaktionskessel ist mit einem Kühler mit Kühlkreislaufpumpe versehen. Das den Reaktionskessel verlassende Reaktionsgemisch wird in einen Phasentrennapparat (Aminalabscheider, 2000) geführt **(Schritt (A-I)).**

Nach der Phasentrennung zur Entfernung der wässrigen Phase (6) wird die organische Phase (5) in einer Mischdüse mit 30%iger wässriger Salzsäure (7) versetzt (Protonierungsgrad 10 %, d. h., pro Mol Aminogruppen werden 0,10 Mol HCl zugegeben) und in den ersten Umlagerungsreaktor (3000-1) gefahren. Der erste Umlagerungsreaktor (sog. "Vakuumkessel") wird bei 50,0 °C betrieben, was mittels einer Siedekühlung an einem Rückflusskühler bei einem Druck von 104 mbar (absolut) sichergestellt wird. Der Rückflusskühler wird mit 0,50 t/h frischem Anilin beaufschlagt. Die Umlagerungsreaktion wird in einer Reaktorkaskade aus insgesamt sieben Reaktoren bei 50,0 °C bis 156,0 °C (d. h. **50,0 °C in Reaktor 3000-1 / 60,0 °C in Reaktor 3000-2 / 83,0 °C in Reaktor 3000-3/ 104,0 °C in Reaktor 3000-4 / 119,0 °C in Reaktor 3000-5 / 148,0 °C in Reaktor 3000-6 / 156,0 °C in Reaktor 3000-7)** zu Ende geführt **(Schritt (A-II)).**

Nach vollständiger Reaktion wird das erhaltene Reaktionsgemisch (8-i) mit 32%iger Natronlauge im molaren Verhältnis von 1,10 : 1 Natronlauge zu HCl versetzt und in einem Neutralisationsrührbehälter (4000) umgesetzt **(Schritt (B-I)).** Die Temperatur beträgt dabei 115,0 °C. Der absolute Druck beträgt 1,40 bar. Die neutralisierte Reaktionsmischung (10) wird anschließend in einem Neutralisationsabscheider (5000) in eine wässrige, untere Phase (12), die zu einem Abwassersammelbehälter geführt wird, und in eine organische Phase (11) getrennt **(Schritt (B-II)).**

Die organische, obere Phase (11) wird zur Wäsche geleitet und in einem gerührten Waschbehälter (6000) mit Kondensat (13) gewaschen **(Schritt (B-III)).** Nach Abtrennung des Waschwassers (15) aus dem im Waschbehälter (6000) erhaltenen zweiphasigen Gemisch (14) in einem Waschwasserabscheider (7000, **Schritt (B-IV))** wird das so erhaltene Roh-MDA (16) durch Destillation von Wasser und Anilin (gemeinsam abgetrennt als Strom 17) befreit, wobei als Sumpfprodukt 17,00 t/h MDA (18) anfielen **(Schritt (B-V)).**

So hergestelltes MDA hat im Mittel eine Zusammensetzung von **45,2 % 4,4'-MDA, 5,5 % 2,4'-MDA, 0,3 % 2,2'-MDA,** also einen **Gesamtzweikerngehalt von 51,0 %** sowie 0,3 % N-Methyl-4,4'-MDA und 0,3 % N-Formyl-4,4'-MDA, wobei der Rest zu 100 % im Wesentlichen aus höheren Homologen (PMDA) und deren Isomeren besteht.

### II. Angestrebter Endzustand: Produktion bei Halblast

**Beispiel 1 (Vergleichsbeispiel): Lastreduzierung der MDA-Anlage von Nennlast auf Halblast (= 50 % der Nennlast), wobei das n(1)/n(2)-Verhältnis und das n(7)/n(1)-Verhältnis gleichgehalten werden, die Einsatzstoffe Anilin, Formalin, Salzsäure und Natronlauge gleichzeitig reduziert werden und die Temperaturen im Vakuumkessel (3000-1), den Folgereaktoren und die Austrittstemperatur der MDA-Rohlösung im letzten Umlagerungsreaktor (3000-7) gleich bleiben**

Die MDA-Anlage wird, wie zuvor unter A.I beschrieben, bei einer Produktionskapazität von 17,00 t/h MDA betrieben. Nun soll der Zweikerngehalt auf ca. 62 % erhöht werden, und zwar verbunden mit einer Halbierung der Last aufgrund geringeren Produktbedarfes. Dazu werden zeitgleich die Einsatzmengen an Anilin und Formalin zum Aminalreaktor (1000) innerhalb von 120 Minuten an die neue Produktionslast angepasst. Die Formalinmenge wird auf 4,80 t/h reduziert.

Die Einsatzanilinmenge wird auf 11,35 t/h reduziert. Gleichzeitig wird der Mengenstrom an Salzsäure in die Mischdüse im Zulauf des ersten Umlagerungsreaktors (3000-1) halbiert. Der erste Umlagerungsreaktor wird weiterhin bei 50,0 °C betrieben, was mittels der Siedekühlung am Rückflusskühler bei 104 mbar (absolut) sichergestellt wird. Der Rückflusskühler wird weiterhin mit 0,50 t/h frischem Anilin beaufschlagt. Die Umlagerungsreaktion wird in einer Reaktorkaskade bei 50,0 °C bis 156,0 °C **(50,0 °C in Reaktor 3000-1 / 60,0 °C in Reaktor 3000-2 / 83,0 °C in Reaktor 3000-3 / 104,0 °C in Reaktor 3000-4 / 119,0 °C in Reaktor 3000-5 / 148,0 °C in Reaktor 3000-6 / 156,0 °C in Reaktor 3000-7)** zu Ende geführt. Nach vollständiger Reaktion wird das erhaltene Reaktionsgemisch wie in den allgemeinen Bedingungen zur Herstellung von MDA beschrieben mit Natronlauge neutralisiert, wobei die Menge an Natronlauge im gleichen Zeitfenster wie Formalin und Anilin reduziert wird, und anschließend zu MDA (18) aufgearbeitet.

40 Stunden nach begonnenem Lastwechsel hat das MDA im Zulauf zum MDA-Tank eine Zusammensetzung von **44,7% 4,4'-MDA, 5,2 % 2,4'-MDA, 0,2 % 2,2'-MDA,** also **einen Gesamtzweikerngehalt von 50,1 %** sowie 0,3 % N-Methyl-4,4'-MDA und 0,3 % N-Formyl-4,4'-MDA, wobei der Rest zu 100 % im Wesentlichen aus höheren Homologen (PMDA) und deren Isomeren besteht. Das MDA weist einen **erhöhten Anteil an unerwünschten Nebenprodukten**

**mit Acridin- und Acridan-Struktur** auf. Siehe hierzu auch Abschnitt **C** weiter unten. Der Zweikerngehalt ist im Wesentlichen gleich dem im Ausgangszustand.

**Beispiel 2 (erfindungsgemäß): Lastreduzierung der MDA-Anlage von Nennlast auf reduzierte Last, wobei das n(1)/n(2)-Verhältnis erhöht wird, die Einsatzstoffe Anilin und Formalin gleichzeitig reduziert werden und wobei das n(7)/n(1)-Verhältnis gleichgehalten wird, Salzsäure und Natronlauge gleichzeitig mit den Einsatzstoffen Anilin und Formalin reduziert werden, die Temperatur im Vakuumkessel (3000-1) gleich bleibt und die Austrittstemperatur der MDA-Rohlösung im letzten Umlagerungsreaktor (3000-7) erniedrigt ist**

Die MDA-Anlage wird, wie zuvor unter A.I beschrieben, bei einer Produktionskapazität von 17,00 t/h MDA betrieben. Nun soll der Zweikerngehalt auf ca. 62 % erhöht werden, und zwar verbunden mit einer Reduzierung der Last auf etwas weniger als die Hälfte der Last im Ausgangszustand aufgrund geringeren Produktbedarfs. Dazu werden zeitgleich die Einsatzmengen an Anilin und Formalin zum Aminalreaktor (1000) innerhalb von 120 Minuten an die neue Produktionslast angepasst. Die Formalinmenge wird auf 4,60 t/h reduziert. Die Einsatzanilinmenge wird auf 14,60 t/h reduziert. Der Mengenstrom an Salzsäure in die Mischdüse im Zulauf des ersten Umlagerungsreaktors wird im gleichen Zeitraum wie Anilin und Formalin unter **Beibehaltung des Protonierungsgrades von 10 %** angepasst. Der erste Umlagerungsreaktor (3000-1) wird weiterhin bei 50,0 °C betrieben, was mittels der Siedekühlung am Rückflusskühler bei 104 mbar (absolut) sichergestellt wird. Der Rückflusskühler wird weiterhin mit 0,50 t/h frischem Anilin beaufschlagt. Die Umlagerungsreaktion wird in einer Reaktorkaskade bei **50,0 °C bis 146,0 °C (50,0 °C in Reaktor 3000-1 / 60,0 °C in Reaktor 3000-2 / 81,0 °C in Reaktor 3000-3 / 95,0 °C in Reaktor 3000-4 / 115,0 °C in Reaktor 3000-5 / 143,0 °C in Reaktor 3000-6 / 146,0 °C in Reaktor 3000-7)** zu Ende geführt (Schritt (A-II)). Nach vollständiger Reaktion wird das erhaltene Reaktionsgemisch wie in den allgemeinen Bedingungen zur Herstellung von MDA beschrieben mit Natronlauge neutralisiert, wobei die Menge an Natronlauge im gleichen Zeitfenster wie Formalin und Anilin und HCl unter Beibehaltung des molaren Verhältnisses von 1,10 : 1 Natronlauge zu HCl reduziert wird, und anschließend zum gewünschten MDA-Typ aufgearbeitet, wobei als Sumpfprodukt der Destillation 8,50 t/h MDA (18) anfallen.

40 Stunden nach begonnenem Lastwechsel hat das MDA im Zulauf zum MDA-Tank eine Zusammensetzung von **54,3 % 4,4'-MDA, 7,5 % 2,4'-MDA, 0,3 % 2,2'-MDA,** also einen **Gesamtzweikerngehalt von 62,1 %** sowie 0,2 % N-Methyl-4,4'-MDA und 0,2 % N-Formyl-4,4'-MDA, wobei der Rest zu 100 % im Wesentlichen aus höheren Homologen (PMDA) und deren Isomeren besteht. Das Produkt unterscheidet entspricht dem gewünschten Zielprodukt. Ein erhöhter Anteil an unerwünschten Nebenprodukten mit Acridin- und Acridan-Struktur wird **nicht** gebildet.

Nachfolgende Tabelle 1 stellt die Resultate aus Abschnitt A einander gegenüber.

| **Tabelle 1: Gegenüberstellung der Beispiele aus Abschnitt A** | Aus gangszustand | Endzustand | Endzustand |
|---|---|---|---|
| | Nennlast | Halblast Beispiel 1 (VgL) | Reduzierte Last Beispiel 2 (erf.-gem.) |
| Anilin (90%ig) in Reaktor 1000 [t/h] | 23,20 | 11,35 | 14,60 |
| Anilin in Reaktor 3000-1 [t/h] | 0,50 | 0,50 | 0,50 |
| Formalin (32%ig) in Reaktor 1000 [t/h] | 9,60 | 4,80 | 4,60 |
| n(1)/n(2) | 2,25 | 2,25 | 2,99 |
| [n(1)/n(2)*(Ü)* **($t=t_2$)**] / [n(1)/n(2)*(A)*] | - | 1,00 | 1,33 |
| Protonierungsgrad [%] | 10 | 10 | 10 |
| n(7)/n(1) | 0,10 | 0,10 | 0,10 |
| Temp.-gradient in Reaktorkaskade 3000 [°C] | 50,0 → 156,0 | 50,0 → 156,0 | 50,0 → 146,0 |
| T(3000-1) [°C] | 50,0 | 50,0 | 50,0 |
| T(3000-2) [°C] | 60,0 | 60,0 | 60,0 |

(fortgesetzt)

| **Tabelle 1: Gegenüberstellung der Beispiele aus Abschnitt A** | Aus gangszustand | Endzustand | Endzustand |
|---|---|---|---|
| | Nennlast | Halblast Beispiel 1 (VgL) | Reduzierte Last Beispiel 2 (erf.-gem.) |
| T(3000-3) [°C] | 83,0 | 83,0 | 81,0 |
| T(3000-4) [°C] | 104,0 | 104,0 | 95,0 |
| T(3000-5) [°C] | 119,0 | 119,0 | 115,0 |
| T(3000-6) [°C] | 148,0 | 148,0 | 143,0 |
| T(3000-7) [°C] | 156,0 | 156,0 | 146,0 |
| Produktionskapazität [t/h] | 17,00 | 8,50 | 8,50 |
| 4,4'-MDA [%] | 45,2 | 44,7 | 54,3 |
| 2,4'-MDA [%] | 5,5 | 5,2 | 7,5 |
| 2,2'-MDA [%] | 0,3 | 0,2 | 0,3 |
| N-Methyl-MDA [%] | 0,3 | 0,3 | 0,2 |
| N-Formyl-MDA [%] | 0,3 | 0,3 | 0,2 |
| Zweikerngehalt [%] | 51,0 | 50,1 | 62,1 |
| Kommentar | - | *Erhöhter Anteil an unerwünschten Nebenprodukten mit Acridin- und Acridan-Struktur; Zweikerngehalt nahezu unverändert* | *Produkt hat wie angestrebt einen erhöhten Zweikerngehalt und ein ähnliches Nebenproduktspektrum wie das Produkt im Ausgangszustand* |

**B. Laststeigerung ausgehend von Produktion mit Halblast**

**I. Ausgangszustand: Beschreibung der für die Herstellung von MDA bei Halblast gewählten Bedingungen**

(Anmerkung: Es gibt selbstverständlich verschiedene Möglichkeiten, eine Produktionsanlage 10 000 bei Halblast zu betreiben. Die in Beispiel 2 eingestellten Bedingungen sind *eine* Möglichkeit; für die folgenden Beispiele 3 und 4 wurde für den Ausgangszustand "Halblast" *eine andere* Möglichkeit gewählt.)

Die Reaktion wird betrieben wie zuvor unter A.I für Nennlast beschrieben mit folgenden Unterschieden:

11,35 t/h Einsatzanilin (enthaltend 90,0 Massen-% Anilin);

4,80 t/h 32%ige wässrige Formaldehydlösung (d. h. das molare Verhältnis von Anilin : Formaldehyd beträgt 2,25 : 1);

**50,0 °C in Reaktor 3000-1 / 60,0 °C in Reaktor 3000-2 / 81,0 °C in Reaktor 3000-3 / 95,0 °C in Reaktor 3000-4 / 116,0 °C in Reaktor 3000-5 / 144,0 °C in Reaktor 3000-6 / 146,0 °C in Reaktor 3000-7;**

Sumpfprodukt von 8,50 t/h MDA (18).

So hergestelltes MDA hat im Mittel eine Zusammensetzung von **46,3 % 4,4'-MDA, 5,0 % 2,4'-MDA, 0,2 % 2,2'-MDA,** also **einen Gesamtzweikerngehalt von 51,5 %** sowie 0,3 % N-Methyl-4,4'-MDA und 0,3 % N-Formyl-4,4'-MDA, wobei der Rest zu 100 % im Wesentlichen aus höheren Homologen (PMDA) und deren Isomeren besteht.

## II. Angestrebter Endzustand: Produktion bei Nennlast

### Beispiel 3 (Vergleichsbeispiel): Lasterhöhung der MDA-Anlage von Halblast auf Nennlast (Nennlast bezogen auf ein MDA mit 62 % Zweikerngehalt), wobei das n(1)/n(2)-Verhältnis erhöht und das n(7)/n(1)-Verhältnis gleichgehalten wird, die Einsatzstoffe Anilin, Formalin, Salzsäure und Natronlauge gleichzeitig erhöht werden und die Temperaturen im Vakuumkessel (3000-1), den Folgereaktoren und die Austrittstemperatur der MDA-Rohlösung im letzten Umlagerungsreaktor (3000-7) gleich bleiben

Die MDA-Anlage wird, wie zuvor unter B.I beschrieben, bei einer Produktionskapazität von 8,50 t/h MDA betrieben. Nun soll der Zweikerngehalt auf ca. 62 % erhöht werden, und zwar verbunden mit einer Erhöhung der Last auf die für ein solches Produkt mögliche Nennlast von $m_2$ = 7,20 t/h aufgrund höheren Produktbedarfs. Dazu werden zeitgleich die Einsatzmengen an Anilin und Formalin zum Aminalreaktor innerhalb von 120 Minuten an die neue Produktionslast angepasst. Die Formalinmenge wird auf 7,20 t/h erhöht. Die Einsatzanilinmenge wird auf 23,20 t/h erhöht. Der Mengenstrom an Salzsäure in die Mischdüse im Zulauf des ersten Umlagerungsreaktors wird im gleichen Zeitraum wie Anilin und Formalin **unter Beibehaltung des Protonierungsgrades von 10 %** (d. h. unter Beibehaltung des n(7)/n(1)-Verhältnisses) erhöht. Der erste Umlagerungsreaktor (3000-1) wird weiterhin bei 50,0 °C betrieben, was mittels der Siedekühlung am Rückflusskühler bei 104 mbar (absolut) sichergestellt wird. Der Rückflusskühler wird mit 0,50 t/h frischem Anilin beaufschlagt. Die Umlagerungsreaktion wird in der Reaktorkaskade weiterhin bei 50,0 °C bis 146 °C **(50,0 °C in Reaktor 3000-1 / 60,0 °C in Reaktor 3000-2 / 82,0 °C in Reaktor 3000-3 / 94,0 °C in Reaktor 3000-4 / 116,0 °C in Reaktor 3000-5 / 144,0 °C in Reaktor 3000-6 / 146,0 °C in Reaktor 3000-7)** zu Ende geführt.

Nach der Reaktion wird das erhaltene Reaktionsgemisch mit 32%iger Natronlauge im molaren Verhältnis von 1,10 : 1 Natronlauge zu HCl versetzt und in einem Neutralisationsrührbehälter umgesetzt, wobei die Menge an Natronlauge im gleichen Zeitfenster wie Formalin, Anilin und HCl unter Beibehaltung der molaren Verhältnisse erhöht wurde. Die weitere Aufarbeitung erfolgt wie oben unter A.I beschrieben. Am Ende des Übergangszustandes werden 13,3 t/h eines Sumpfproduktes erhalten.

Ergebnis: So hergestelltes "MDA" ist nicht vollständig umgelagert und enthält noch teilumgelagerte Produkte wie Aminobenzylaniline, die zu Qualitätsproblemen bei der sich anschließenden Phosgenierung zu MDI führen, wie z. B. deutlich erhöhte Farbwerte im resultierenden MDI-Produkt. Das so hergestellte MDA als auch das daraus resultierende MDI sind nicht spezifikationsgerechte Ware.

### Beispiel 4 (erfindungsgemäß):

### Lasterhöhung der MDA-Anlage von Halblast auf Nennlast (Nennlast bezogen auf ein MDA mit 62 % Zweikerngehalt), wobei das n(1)/n(2)-Verhältnis erhöht wird, die Einsatzstoffe Anilin und Formalin gleichzeitig erhöht werden und wobei das n(7)/n(1)-Verhältnis gleich gehalten wird, Salzsäure und Natronlauge gleichzeitig erhöht werden, die Temperatur im Vakuumkessel (3000-1) gleichgehalten wird und die Austrittstemperatur der MDA-Rohlösung im letzten Umlagerungsreaktor (3000-7) erhöht wird

Die MDA-Anlage wird, wie zuvor unter B.I beschrieben, bei einer Produktionskapazität von 8,50 t/h MDA betrieben. Nun soll der Zweikerngehalt des Produkts auf ca. 62 % erhöht werden, wobei dieses geänderte Produkt aufgrund höheren Produktbedarfs mit der ihm eigenen maximal möglichen Produktionskapazität hergestellt werden soll. Dazu werden zeitgleich die Einsatzmengen an Anilin und Formalin zum Aminalreaktor innerhalb von 120 Minuten an die neue Produktionslast angepasst. Die Formalinmenge wird auf 7,20 t/h erhöht. Die Einsatzanilinmenge wird auf 23,20 t/h erhöht. Der Mengenstrom an Salzsäure in die Mischdüse im Zulauf des ersten Umlagerungsreaktors wird im gleichen Zeitraum wie Anilin und Formalin **unter Beibehaltung des Protonierungsgrades von 10 %** angepasst. Der erste Umlagerungsreaktor (3000-1) wird weiterhin bei 50,0 °C betrieben, was mittels der Siedekühlung am Rückflusskühler bei 104 mbar (absolut) sichergestellt wird. Der Rückflusskühler wird mit 0,50 t/h frischem Anilin beaufschlagt. Die Umlagerungsreaktion wird in der Reaktorkaskade bei 50,0 °C bis 156 °C **(50,0 °C in Reaktor 3000-1 / 60,0 °C in Reaktor 3000-2 / 83,0 °C in Reaktor 3000-3 / 104,0 °C in Reaktor 3000-4 / 119,0 °C in Reaktor 3000-5 / 148,0 °C in Reaktor 3000-6 / 156,0 °C in Reaktor 3000-7)** zu Ende geführt **(Schritt A-II))**.

Nach der Reaktion wird das erhaltene Reaktionsgemisch mit 32%iger Natronlauge im molaren Verhältnis von 1,10 : 1 Natronlauge zu HCl versetzt und in einem Neutralisationsrührbehälter umgesetzt, wobei die Menge an Natronlauge im gleichen Zeitfenster wie Formalin, Anilin und Salzsäure erhöht wurde. Die Temperatur beträgt 115,0 °C. Der absolute Druck beträgt 1,40 bar. Die weitere Aufarbeitung erfolgt wie weiter oben unter A.I beschrieben. Am Ende des Übergangszustandes werden als Sumpfprodukt 13,30 t/h MDA (18) erhalten.

20 Stunden nach begonnenem Lastwechsel hat das MDA im Zulauf zum MDA-Tank eine Zusammensetzung von **53,6 % 4,4'-MDA, 7,8 % 2,4'-MDA, 0,4 % 2,2'-MDA,** also einen **Gesamtzweikerngehalt von 61,8 %** sowie 0,2 % N-Methyl-4,4'-MDA und 0,3 % N-Formyl-4,4'-MDA, wobei der Rest zu 100 % im Wesentlichen aus höheren Homologen

(PMDA) und deren Isomeren besteht. Das Produkt unterscheidet sich nur unwesentlich vom MDA-Strom (18), der im Ausgangszustand wie unter B.I beschrieben im Mittel erhalten wird. Ein erhöhter Anteil an unerwünschten Nebenprodukten mit Acridin- und Acridan-Struktur wird **nicht** gebildet.

Nachfolgende Tabelle 2 stellt die Resultate aus Abschnitt B einander gegenüber.

| **Tabelle 2: Gegenüberstellung der Beispiele aus Abschnitt B** | Aus gangs zustand | Endzustand | Endzustand |
|---|---|---|---|
| | Halblast | Nennlast des Zielprodukts Beispiel 3 (Vgl.) | Nennlast des Zielprodukts Beispiel 4 (erf.-gem.) |
| Anilin (90%ig) in Reaktor 1000 [t/h] | 11,35 | 23,20 | 23,20 |
| Anilin in Reaktor 3000-1 [t/h] | 0,50 | 0,50 | 0,50 |
| Formalin (32%ig) in Reaktor 1000 [t/h] | 4,80 | 7,20 | 7,20 |
| n(1)/n(2) | 2,25 | 2,99 | 2,99 |
| [n(1)/n(2)*(Ü)***(t=$t_2$)**] / [n(1)/n(2)*(A)*] | - | 1,33 | 1,33 |
| Protonierungsgrad [%] | 10 | 10 | 10 |
| n(7)/n(1) | 0,10 | 0,10 | 0,10 |
| Temp.-gradient in Reaktorkaskade 3000 [°C] | 50,0 → 146,0 | 50,0 → 146,0 | 50,0 → 156,0 |
| T(3000-1) [°C] | 50,0 | 50,0 | 50,0 |
| T(3000-2) [°C] | 60,0 | 60,0 | 60,0 |
| T(3000-3) [°C] | 81,0 | 82,0 | 83,0 |
| T(3000-4) [°C] | 95,0 | 94,0 | 104,0 |
| T(3000-5) [°C] | 116,0 | 116,0 | 119,0 |
| T(3000-6) [°C] | 144,0 | 144,0 | 148,0 |
| T(3000-7) [°C] | 146,0 | 146,0 | 156,0 |
| Produktionskapazität [t/h] | 8,50 | 13,30 | 13,30 |
| 4,4'-MDA [%] | 46,3 | - | 53,6 |
| 2,4'-MDA [%] | 5,0 | - | 7,8 |
| 2,2'-MDA [%] | 0,2 | - | 0,4 |
| N-Methyl-MDA [%] | 0,3 | - | 0,2 |
| N-Formvl-MDA [%] | 0,3 | - | 0,3 |
| Zweikerngehalt [%] | 51,5 | - | 61,8 |
| Kommentar | - | *"MDA" ist nicht vollständig umgelagert und enthält noch teilumgelagerte Produkte wie Aminobenzylaniline, die zu Qualitätsprobleme n bei der sich anschließenden Phosgenierung zu MDI führen, wie z. B. deutlich erhöhte Farbwerte* | *Produkt hat wie angestrebt einen erhöhten Zweikerngehalt und ein ähnliches Nebenproduktspektrum wie das Produkt im Ausgangszustand* |

## C. Prinzipversuche zur Bildung von Nebenprodukten mit Acridin- und Acridan-Struktur

In einem Reihenversuch wurde die jeweils benötigte Menge an wässriger 30%iger Salzsäure zur Erzielung des gewünschten Protonierungsgrades (siehe nachfolgende Tabelle 3) zu einer auf 100,0 °C vortemperierten 2,2'-MDA-Lösung in Anilin zugegeben. Die 2,2'-MDA-Konzentrationen in den einzelnen Versuchen betrug jeweils 1,0 Massen%; zusätzlich enthielten die Lösungen Octadecan als internen Standard für die gaschromatographische (GC) Analyse. Das resultierende Gemisch wurde schnellstmöglich mittels einer peristaltischen Pumpe in einen auf 120,0 °C vorgeheizten Büchi-Glasautoklaven überführt und auf die vorgesehene Reaktionstemperatur erhitzt (siehe Tabelle 3). Beim Erreichen der angestrebten Reaktionstemperatur wurde die erste Probe gezogen (Zeit gleich Null). Um den Reaktionsfortschritt zu verfolgen, wurden weitere Proben nach 30, 60, 120 und 240 Minuten gezogen und mittels GC-Analyse untersucht. Reaktionsbedingungen und Versuchsergebnisse sind in der nachfolgenden Tabelle 3 zusammengefasst.

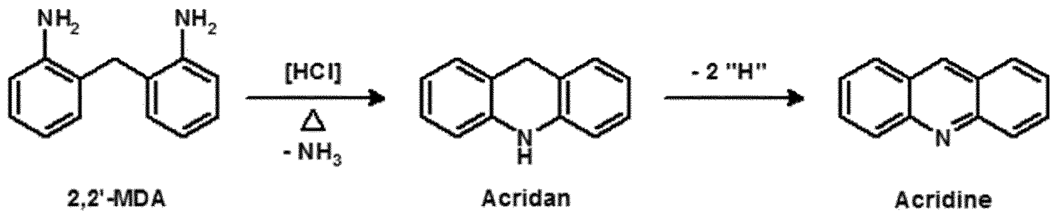


Schema 1: Entstehung von Acridan und Acridin aus 2,2´-MDA

Tabelle 3: Laborversuche zur Entstehung der Nebenkomponenten Acridan und Acridin

| Versuch | Reaktionstemperatur [°C] | Protonierungsgrad [%] | Zeit [min] | Summe (Acridin+Acridan) [ppm] |
|---|---|---|---|---|
| **1** | 160 °C | 10% | 15 | 79 |
| | | | 30 | 208 |
| | | | 60 | 421 |
| | | | 120 | 925 |
| | | | 240 | 1775 |
| **2** | 170 °C | 25% | 0 | 977 |
| | | | 30 | 2554 |
| | | | 60 | 3950 |
| | | | 120 | 5647 |
| | | | 240 | 8894 |
| **3** | 160 °C | 25% | 0 | 364 |
| | | | 30 | 930 |
| | | | 60 | 1594 |
| | | | 120 | 2681 |
| | | | 240 | 4354 |
| **4** | 180 | 10% | 0 | 606 |
| | | | 30 | 1630 |
| | | | 60 | 2613 |
| | | | 120 | 3932 |
| | | | 240 | 5294 |
| **5** | 170 | 10% | 0 | 292 |

(fortgesetzt)

| Versuch | Reaktionstemperatur [°C] | Protonierungsgrad [%] | Zeit [min] | Summe (Acridin+Acridan) [ppm] |
|---|---|---|---|---|
| | | | 30 | 794 |
| | | | 60 | 1533 |
| | | | 120 | 2406 |
| | | | 240 | 3932 |
| **6** | 160 | 5% | 0 | 0 |
| | | | 30 | 0 |
| | | | 60 | 0 |
| | | | 120 | 0 |
| | | | 240 | 0 |
| **7** | 170 | 5% | 0 | 0 |
| | | | 30 | 0 |
| | | | 60 | 0 |
| | | | 120 | 0 |
| | | | 240 | 218 |
| **8** | 180 | 5% | 0 | 0 |
| | | | 30 | 402 |
| | | | 60 | 834 |
| | | | 120 | 1355 |
| | | | 240 | 2380 |

Es zeigt sich, dass mit steigender Temperatur und steigender Salzsäurekonzentration die Bildung der Nebenkomponenten Acridin und Acridan aus 2,2'-MDA vermehrt auftritt.

**Patentansprüche**

**1.** Verfahren zur Herstellung von Di- und Polyaminen der Diphenylmethanreihe aus Anilin (1) und Formaldehyd (2) in einer Produktionsanlage (10 000), wobei das molare Verhältnis von insgesamt eingesetztem Anilin (1) zu insgesamt eingesetztem Formaldehyd (2), n(1)/n(2), stets gleich oder größer als 1,6 ist, umfassend die Schritte:

(A-I) Umsetzung von Anilin (1) und Formaldehyd (2) in Abwesenheit eines sauren Katalysators unter Erhalt eines Aminal (3) enthaltenden Reaktionsgemisches (4), und anschließende mindestens teilweise Abtrennung einer wässrigen Phase (6) aus dem Reaktionsgemisch (4) unter Erhalt einer organischen, das Aminal (3) enthaltenden Phase (5);
(A-II) Inkontaktbringen der in Schritt (A-I) erhaltenen organischen, das Aminal enthaltenden Phase (5) mit einem sauren Katalysator (7) in einer Reaktorkaskade (3000) aus i in Serie geschalteten Reaktoren (3000-1, 3000-2, ..., 3000-i), wobei i eine natürliche Zahl von 2 bis 10 ist, wobei

• der in Strömungsrichtung erste Reaktor (3000-1) bei einer Temperatur $T_{3000-1}$ im Bereich von 25,0 °C bis 65,0 °C betrieben wird und mit dem Strom (5) und saurem Katalysator (7) sowie optional mit weiterem Anilin (1) und/oder weiterem Formaldehyd (2) beschickt wird,
• jeder in Strömungsrichtung nachfolgende Reaktor (3000-2, ..., 3000-i) bei einer Temperatur von mehr als 2,0 °C oberhalb von $T_{3000-1}$ betrieben wird und mit dem im unmittelbar vorangehenden Reaktor erhaltenen Reaktionsgemisch beschickt wird;

(B) Isolierung der Di- und Polyamine der Diphenylmethanreihe aus dem im letzten Reaktor (3000-i) aus Schritt

(A-II) erhaltenen Reaktionsgemisch (8-i) durch mindestens

(B-I) Zugabe eines bezogen auf die insgesamt eingesetzte Menge an saurem Katalysator (7) stöchiometrischen Überschusses an Base (9) zu dem in Schritt (A-II) im letzten Reaktor (3000-i) erhaltenen Reaktionsgemisch (8-i) unter Erhalt eines Reaktionsgemisches (10);

(B-II) Auftrennen des in Schritt (B-I) erhaltenen Reaktionsgemisches (10) in eine organische Phase (11), umfassend Di- und Polyamine der Diphenylmethanreihe und eine wässrige Phase (12);

wobei

bei einer Änderung der Produktionskapazität von einem Ausgangszustand *A* mit

- einem Massenstrom $m_1$ an insgesamt eingesetztem Anilin im Ausgangszustand von $m_1(A) \neq 0$,
- einem Massenstrom $m_2$ an insgesamt eingesetztem Formaldehyd im Ausgangszustand von $m_2(A) = X(A) \cdot m_2(N)$, wobei $X(A)$ eine dimensionslose Zahl > 0 und ≤ 1 ist und $m_2(N)$ die Nennlast der Produktionsanlage (10 000) bezeichnet,
- einem molaren Verhältnis n(1)/n(2) von insgesamt eingesetztem Anilin (1) zu insgesamt eingesetztem Formaldehyd (2) im Ausgangszustand von n(1)/n(2)*(A)*,
- einem molaren Verhältnis n(7)/n(1) von insgesamt eingesetztem saurem Katalysator zu insgesamt eingesetztem Anilin im Ausgangszustand von n(7)/n(1)*(A)* und
- einem auf die Gesamtmasse an Di- und Polyaminen der Diphenylmethanreihe bezogenen Massenanteil $\omega_{MMDA}$ an Diaminen der Diphenylmethanreihe von $\omega_{MMDA}(A)$ in einen Endzustand *E* mit
- einem Massenstrom $m_1$ an insgesamt eingesetztem Anilin von im Endzustand $m_1(E) \neq 0$,
- einem Massenstrom $m_2$ an insgesamt eingesetztem Formaldehyd im Endzustand von $m_2(E) = X(E) \cdot m_2(N)$, wobei $X(E)$ eine dimensionslose Zahl > 0 und ≤ 1 ist,
- einem molaren Verhältnis n(1)/n(2) von insgesamt eingesetztem Anilin (1) zu insgesamt eingesetztem Formaldehyd (2) im Endzustand von n(1)/n(2)*(E)*,
- einem molaren Verhältnis n(7)/n(1) von insgesamt eingesetztem saurem Katalysator zu insgesamt eingesetztem Anilin im Endzustand von n(7)/n(1)*(E)* und
- einem auf die Gesamtmasse an Di- und Polyaminen der Diphenylmethanreihe bezogenen Massenanteil $\omega_{MMDA}$ an Diaminen der Diphenylmethanreihe, der für den Endzustand angestrebt ist, von $\omega_{MMDA}(E)$;

um einen Betrag $\Delta X = |X(E) - X(A)|$, mit $\Delta X \geq 0{,}10$, wobei das Verfahren mindestens eine so definierte Änderung der Produktionskapazität umfasst, wobei diese Änderung der Produktionskapazität bei einem Zeitpunkt $t_1$ beginnt und bei einem Zeitpunkt $t_2$ abgeschlossen ist, wobei weiterhin $\omega_{MMDA}$ so geändert wird, dass der für den Endzustand angestrebte Wert $\omega_{MMDA}(E)$ entweder $\geq 1{,}05 \cdot \omega_{MMDA}(A)$ oder $\leq 0{,}95 \cdot \omega_{MMDA}(A)$ ist,

und wobei gilt:

$0{,}90 \cdot n(7)/n(1)(A) \leq n(7)/n(1)(E) \leq 1{,}10 \cdot n(7)/n(1)(A)$;

**dadurch gekennzeichnet, dass** im Zeitraum von $t_1$ bis $t_2$, dem Übergangszustand *Ü*, mit einem molaren Verhältnis von insgesamt eingesetztem Anilin (1) zu insgesamt eingesetztem Formaldehyd (2) von n(1)/n(2)*(Ü)* und einem molaren Verhältnis von insgesamt eingesetztem saurem Katalysator zu insgesamt eingesetztem Anilin von n(7)/n(1)*(Ü)*

(i) die Temperatur im in Strömungsrichtung ersten Reaktor (3000-1) aus Schritt (A-II) auf einen Wert eingestellt wird, der von der Temperatur in diesem Reaktor während des Ausgangszustands *A* um maximal 10,0 °C abweicht;

(ii-1) im Fall von $m_2(E) > m_2(A)$, die Temperatur in mindestens einem der in Strömungsrichtung nachfolgenden Reaktoren (3000-2, ..., 3000-i) im Vergleich zum Ausgangszustand *A* so um mehr als 2,0 °C erhöht wird, dass die angestrebte Endtemperatur spätestens zum Zeitpunkt $t_2$ vorliegt, und in allen Reaktoren (3000-2, ..., 3000-i), in denen die Temperatur nicht erhöht wird, im Bereich einer Schwankungsbreite von ± 2,0 °C gleich gehalten wird;

(ii-2) im Fall von $m_2(E) < m_2(A)$, die Temperatur in mindestens einem der in Strömungsrichtung nachfolgenden Reaktoren (3000-2, ..., 3000-i) im Vergleich zum Ausgangszustand *A* so um mehr als 2,0 °C erniedrigt wird, dass die angestrebte Endtemperatur spätestens zum Zeitpunkt $t_2$ vorliegt und in allen Reaktoren (3000-2, ..., 3000-i), in denen die Temperatur nicht erniedrigt wird, im Bereich einer Schwankungsbreite von ± 2,0 °C gleich gehalten wird;

(iii-1) im Fall von $\omega_{MMDA}(E) \geq 1{,}05 \cdot \omega_{MMDA}(A)$, n(1)/n(2)*(Ü)* so eingestellt wird, dass zum Zeitpunkt $t_2$ gilt:

$1{,}05 \cdot n(1)/n(2)(A) \leq n(1)/n(2)(Ü) \leq 2{,}00 \cdot n(1)/n(2)(A)$;

wobei während des gesamten Übergangszustandes vor Erreichen des Zeitpunkts $t_2$ stets gilt:

$0{,}80 \cdot n(1)/n(2)(A) \leq n(1)/n(2)(Ü) \leq 2{,}50 \cdot n(1)/n(2)(A)$;

(iii-2) im Fall von $\omega_{MMDA}(E) \leq 0{,}95 \cdot \omega_{MMDA}(A)$, n(1)/n(2)*(Ü)* so eingestellt wird, dass zum Zeitpunkt $t_2$ gilt:

$0{,}50 \cdot n(1)/n(2)(A) \leq n(1)/n(2)(Ü) \leq 0{,}95 \cdot n(1)/n(2)(A)$
wobei während des gesamten Übergangszustandes vor Erreichen des Zeitpunkts $t_2$ stets gilt:
$0{,}40 \cdot n(1)/n(2)(A) \leq n(1)/n(2)(Ü) \leq 2{,}50 \cdot n(1)/n(2)(A)$.

**2.** Verfahren gemäß Anspruch 1, bei dem die Temperatur in den Reaktoren der Reaktorkaskade 3000 von Reaktor 3000-1 bis Reaktor 3000-i in allen Betriebszuständen (*A*, *Ü, E)* zunimmt.

**3.** Verfahren gemäß einem der Ansprüche 1 oder 2, bei dem stets

• $T_{3000-1}$ auf einen Wert im Bereich von 25,0 °C bis 65,0 °C und
• die Temperatur in allen in Strömungsrichtung nachfolgenden Reaktoren (3000-2, ..., 3000-i) jeweils auf einen Wert im Bereich von 35,0 °C bis 200,0 °C,
eingestellt wird.

**4.** Verfahren gemäß Anspruch 3, bei dem stets

• $T_{3000-1}$ auf einen Wert im Bereich von 30,0 °C bis 60,0 °C und
• die Temperatur in allen in Strömungsrichtung nachfolgenden Reaktoren (3000-2, ..., 3000-i) jeweils auf einen Wert im Bereich von 50,0 °C bis 180,0 °C,
eingestellt wird.

**5.** Verfahren gemäß einem der vorstehenden Ansprüche, bei dem der saure Katalysator (7) eine Mineralsäure ist.

**6.** Verfahren gemäß einem der vorstehenden Ansprüche, bei dem Schritt (B) weiter umfasst:

(B-III) Waschen der organischen Phase (11) mit Waschflüssigkeit (13);
(B-IV) Auftrennen des in Schritt (B-III) erhaltenen Gemisches (14) in eine organische Phase (16) umfassend Di- und Polyamine der Diphenylmethanreihe und eine wässrige Phase (15);
(B-V) Destillieren der organischen Phase (16) aus Schritt (B-IV) unter Erhalt der Di- und Polyamine der Diphenylmethanreihe (18), wobei ein Wasser und Anilin enthaltender Strom (17) abgetrennt wird.

**7.** Verfahren gemäß Anspruch 6, das zusätzlich umfasst:
(C) Rückführung des Stroms (17), ggf. nach Aufarbeitung, in Schritt (A-I) und/oder, sofern die optionale Zugabe weiteren Anilins (1) in Schritt (A-II) durchgeführt wird, in Schritt (A-II).

**8.** Verfahren gemäß einem der vorstehenden Ansprüche, bei dem im Fall von $\omega_{MMDA}(E) \geq 1{,}05 \cdot \omega_{MMDA}(A)$, $m_2(E)$ gegenüber $m_2(A)$ erniedrigt wird.

**9.** Verfahren gemäß einem der vorstehenden Ansprüche, bei dem im Fall von $\omega_{MMDA}(E) \leq 0{,}95 \cdot \omega_{MMDA}(A)$, $m_2(E)$ gegenüber $m_2(A)$ erhöht wird.

**10.** Verfahren gemäß einem der vorstehenden Ansprüche, bei dem das molare Verhältnis von insgesamt eingesetztem Anilin (1) zu insgesamt eingesetztem Formaldehyd (2), n(1)/n(2), in allen Betriebszuständen (*A*, *Ü*, *E*) auf einen Wert von 1,6 bis 20 eingestellt wird.

**11.** Verfahren gemäß einem der vorstehenden Ansprüche, bei dem die Werte für n(1)/n(2), n(7)/n(1) und für die Temperatur eines jeden Reaktors j der Reaktorkaskade (3000), $T_{3000-j}$, welche zum Zeitpunkt $t_2$ jeweils vorliegen, für die Dauer der Produktion mit dem Formaldehyd-Massenstrom $m_2(E)$ beibehalten werden.

**12.** Verfahren gemäß einem der vorstehenden Ansprüche, bei dem während des gesamten Übergangszustandes *Ü* vor Erreichen des Zeitpunkts $t_2$ für das molare Verhältnis von insgesamt eingesetztem saurem Katalysator zu insgesamt eingesetztem Anilin n(7)/n(1)*(Ü)*, gilt:
$0{,}85 \cdot n(7)/n(1)(A) \leq n(7)/n(1)(Ü) \leq 1{,}15 \cdot n(7)/n(1)(A)$.

**13.** Verfahren gemäß Anspruch 10, bei dem zum Zeitpunkt $t_2$ gilt:

$$n(7)/n(1)(E) = n(7)/n(1)(A)).$$

**14.** Verfahren gemäß einem der vorstehenden Ansprüche, bei dem der angestrebte Endwert des molaren Verhältnisses von insgesamt eingesetztem Anilin zu insgesamt eingesetztem Formaldehyd, n(1)/n(2)*(E)*, durch kontinuierliche Anpassung des Werts von $m_2$ und mindestens einem der Werte von $m_1$ und/oder $m_7$ bis zum Zeitpunkt $t_2$ eingestellt wird.

**15.** Verfahren gemäß einem der vorstehenden Ansprüche, bei dem der Zeitraum von $t_1$ bis $t_2$ von 1,00 Minuten bis 120 Minuten dauert.

## Claims

**1.** Process for preparing di- and polyamines of the diphenylmethane series from aniline (1) and formaldehyde (2) in a production plant (10 000), where the molar ratio of total aniline used (1) to total formaldehyde used (2), n(1)/n(2), is always not less than 1.6, comprising the steps of:

(A-I) reacting aniline (1) and formaldehyde (2) in the absence of an acidic catalyst to obtain a reaction mixture (4) comprising aminal (3), and then at least partly separating an aqueous phase (6) from the reaction mixture (4) to obtain an organic phase (5) comprising the aminal (3);
(A-II) contacting the organic phase (5) which comprises the aminal and is obtained in step (A-I) with an acidic catalyst (7) in a reactor cascade (3000) composed of i reactors connected in series (3000-1, 3000-2, ..., 3000-i), where i is a natural number from 2 to 10, wherein

- the first reactor (3000-1) in flow direction is operated at a temperature $T_{3000-1}$ in the range from 25.0°C to 65.0°C, and is charged with stream (5) and acidic catalyst (7) and optionally with further aniline (1) and/or further formaldehyde (2),
- every reactor downstream in flow direction (3000-2, ..., 3000-i) is operated at a temperature of more than 2.0°C above $T_{3000-1}$ and is charged with the reaction mixture obtained in the reactor immediately upstream;

(B) isolating the di- and polyamines of the diphenylmethane series from the reaction mixture (8-i) obtained from step (A-II) in the last reactor (3000-i) by at least
(B-I) adding a stoichiometric excess of base (9), based on the total amount of acidic catalyst used (7), to the reaction mixture (8-i) obtained in the last reactor (3000-i) in step (A-II) to obtain a reaction mixture (10);
(B-II) separating the reaction mixture (10) obtained in step (B-I) into an organic phase (11) comprising di- and polyamines of the diphenylmethane series and an aqueous phase (12);
wherein
in the event of a change in the production capacity from a starting state A with

- a mass flow rate $m_1$ of total aniline used in the starting state of $m_1$*(A)* $\neq 0$,
- a mass flow rate $m_2$ of total formaldehyde used in the starting state of $m_2$ *(A)* = X*(A)* · $m_2$*(N),* where *X(A)* is a dimensionless number > 0 and ≤ 1 and $m_2$*(N)* denotes the nameplate load of the production plant (10 000),
- a molar ratio n(1)/n(2) of total aniline used (1) to total formaldehyde used (2) in the starting state of n(1)/n(2) *(A)*,
- a molar ratio n(7)/n(1) of total acidic catalyst used to total aniline used in the starting state of n(7)/n(1) *(A)* and
- a proportion by mass $\omega_{MMDA}$, based on the total mass of di- and polyamines of the diphenylmethane series, of diamines of the diphenylmethane series of $\omega_{MMDA}$*(A)* to an end state *E* with
- a mass flow rate $m_1$ of total aniline used of in the end state $m_1$*(E)* $\neq 0$,
- a mass flow rate $m_2$ of total formaldehyde used in the end state of $m_2$*(E)* = X*(E)* · $m_2$*(N)*, where X*(E)* is a dimensionless number > 0 and ≤ 1,
- a molar ratio n(1)/n(2) of total aniline used (1) to total formaldehyde used (2) in the end state of n(1)/n(2) *(E),*
- a molar ratio n(7)/n(1) of total acidic catalyst used to total aniline used in the end state of n(7)/n(1) *(E)* and
- a target proportion by mass $\omega_{MMDA}$, based on the total mass of di- and polyamines of the diphenylmethane series, of diamines of the diphenylmethane series for the end state of $\omega_{MMDA}$ *(E)*

by a quantity $\Delta X = |X(E) - X(A)|$, with $\Delta X \geq 0.10$, wherein the process comprises at least one change in production capacity as thus defined, wherein this change in the production capacity commences at a time $t_1$ and has concluded at a time $t_2$, wherein $\omega_{MMDA}$ is also altered in such a way that, for the target value of $\omega_{MMDA}$*(E)* for the end state, either $\geq 1.05 \cdot \omega_{MMDA}(A)$ or $\leq 0.95 \cdot \omega_{MMDA}(A)$, and wherein:
$0.90 \cdot n(7)/n(1)\ (A) \leq n(7)/n(1)\ (E) \leq 1.10 \cdot n(7)/n(1)\ (A)$;
**characterized in that,** in the period from $t_1$ to $t_2$, the transition state *T*, with a molar ratio of total aniline

used (1) to total formaldehyde used (2) of n(1)/n(2) *(T)* and a molar ratio of total acidic catalyst used to total aniline used of n(7)/n(1)*(T)*,

(i) the temperature in the first reactor (3000-1) in flow direction from step (A-II) is adjusted to a value that differs from the temperature **in that** reactor during the starting state *A* by not more than 10.0°C;
(ii-1) in the case that $m_2(E) > m_2(A)$, the temperature in at least one of the reactors downstream in flow direction (3000-2, ..., 3000-i), by comparison with the starting state *A*, is increased by more than 2.0°C in such a way that the target end temperature is reached no later than at time $t_2$, and in all reactors (3000-2, ..., 3000-i) in which the temperature is not increased it is kept the same within a range of variation of ± 2.0°C;
(ii-2) in the case that $m_2(E) < m_2(A)$, the temperature in at least one of the reactors downstream in flow direction (3000-2, ..., 3000-i), by comparison with the starting state *A*, is lowered by more than 2.0°C in such a way that the target end temperature is reached no later than at time $t_2$, and in all reactors (3000-2, ..., 3000-i) in which the temperature is not lowered it is kept the same within a range of variation of ± 2.0°C;
(iii-1) in the case that $\omega_{MMDA}(E) \geq 1.05 \cdot \omega_{MMDA}(A)$, n(1)/n(2) *(T)* is adjusted such that, at time $t_2$:
$1.05 \cdot n(1)/n(2)\ (A) \leq n(1)/n(2)\ (T) \leq 2.00 \cdot n(1)/\ n(2)\ (A)$;
where it is always the case throughout the transition state prior to attainment of time $t_2$ that:
$0.80 \cdot n(1)/n(2)\ (A) \leq n(1)/n(2)\ (T) \leq 2.50 \cdot n(1)/\ n(2)\ (A)$;
(iii-2) in the case that $\omega_{MMDA}(E) \leq 0.95 \cdot \omega_{MMDA}(A)$, n(1)/n(2) *(T)* is adjusted such that, at time $t_2$:
$0.50 \cdot n(1)/n(2)\ (A) \leq n(1)/n(2)\ (T) \leq 0.95 \cdot n(1)/\ n(2)\ (A)$
where it is always the case throughout the transition state prior to attainment of time $t_2$ that:
$0.40 \cdot n(1)/n(2)\ (A) \leq n(1)/n(2)\ (T) \leq 2.50 \cdot n(1)/n\ (2)\ (A)$.

**2.** Process according to Claim 1, in which the temperature in the reactors of the reactor cascade 3000 increases from reactor 3000-1 to reactor 3000-i in all states of operation (*A, T*, *E*).

**3.** Process according to either of Claims 1 and 2, in which it is always the case that

• $T_{3000-1}$ is set to a value in the range from 25.0°C to 65.0°C and
• the temperature in each of the reactors downstream in flow direction (3000-2, ..., 3000-i) is set to a value in the range from 35.0°C to 200.0°C.

**4.** Process according to Claim 3, in which it is always the case that

• $T_{3000-1}$ is set to a value in the range from 30.0°C to 60.0°C and
• the temperature in each of the reactors downstream in flow direction (3000-2, ..., 3000-i) is set to a value in the range from 50.0°C to 180.0°C.

**5.** Process according to any of the preceding claims, in which the acidic catalyst (7) is a mineral acid.

**6.** Process according to any of the preceding claims, in which step (B) further comprises:

(B-III) washing the organic phase (11) with washing liquid (13);
(B-IV) separating the mixture (14) obtained in step (B-III) into an organic phase (16) comprising di- and polyamines of the diphenylmethane series and an aqueous phase (15);
(B-V) distilling the organic phase (16) from step (B-IV) to obtain the di- and polyamines of the diphenylmethane series (18), with removal of a stream (17) comprising water and aniline.

**7.** Process according to Claim 6, additionally comprising:
(C) recycling stream (17), optionally after workup, into step (A-I) and/or, if the optional addition of further aniline (1) in step (A-II) is conducted, into step (A-II).

**8.** Process according to any of the preceding claims, in which, in the case that $\omega_{MMDA}(E) \geq 1.05 \cdot \omega_{MMDA}(A)$, $m_2(E)$ is lowered relative to $m_2(A)$.

**9.** Process according to any of the preceding claims, in which, in the case that $\omega_{MMDA}(E) \leq 0.95 \cdot \omega_{MMDA}(A)$, $m_2(E)$ is increased relative to $m_2(A)$.

**10.** Process according to any of the preceding claims, in which the molar ratio of total aniline used (1) to total formaldehyde used (2), n(1)/n(2), in all states of operation (*A*, *T*, *E*) is adjusted to a value of 1.6 to 20.

**11.** Process according to any of the preceding claims, in which the values of n(1)/n(2), n(7)/n(1) and of the temperature of each reactor j of the reactor cascade (3000), $T_{3000\text{-}j}$, that exist in each case at time $t_2$ are retained for the duration of the production with the formaldehyde mass flow rate $m_2(E)$.

**12.** Process according to any of the preceding claims, in which, throughout the transition state *T* prior to attainment of time $t_2$, the following is true of the molar ratio of total acid catalyst used to total aniline used n(7)/n(1) *(T)* : $0.85 \cdot n(7)/n(1)\ (A) \leq n(7)/n(1)\ (T) \leq 1.15 \cdot n(7)/n(1)\ (A)$.

**13.** Process according to Claim 10, in which the following is true at time $t_2$:

$$n(7)/n(1)\ (E) = n(7)/n(1)\ (A).$$

**14.** Process according to any of the preceding claims, in which the target end value for the molar ratio of total aniline used to total formaldehyde used, n(1)/n(2) *(E)* is established by continuously adjusting the value of $m_2$ and at least one of the values of $m_1$ and/or $m_7$ until time $t_2$.

**15.** Process according to any of the preceding claims, in which the period from $t_1$ to $t_2$ lasts from 1.00 minute to 120 minutes.

## Revendications

**1.** Procédé pour la production de di- et polyamines de la série du diphénylméthane à partir d'aniline (1) et de formaldéhyde (2) dans une unité de production (10 000), où le rapport molaire d'aniline (1) utilisée au total au formaldéhyde (2) utilisé au total, n(1)/n(2), est toujours égal ou supérieur à 1,6, comprenant les étapes :

(A-I) mise en réaction d'aniline (1) et de formaldéhyde (2) en absence d'un catalyseur acide avec obtention d'un mélange réactionnel (4) contenant de l'aminal (3), et séparation au moins partielle subséquente d'une phase aqueuse (6) d'avec le mélange réactionnel (4) avec obtention d'une phase organique (5) contenant l'aminal (3) ;
(A-II) mise en contact de la phase organique (5) contenant l'aminal, obtenue dans l'étape (A-I), avec un catalyseur acide (7) dans une cascade de réacteurs (3000) constituée de i réacteurs (3000-1, 3000-2, ..., 3000-i) montés en série, i étant un nombre naturel valant de 2 à 10,
où

- le premier réacteur (3000-1) dans le sens de l'écoulement est conduit à une température $T_{3000\text{-}1}$ dans la plage de 25,0 °C à 65,0 °C et est alimenté avec le flux (5) et le catalyseur acide (7) ainsi qu'en option avec de l'aniline supplémentaire (1) et/ou du formaldéhyde supplémentaire (2),
- chaque réacteur (3000-2, ..., 3000-i) suivant dans le sens de l'écoulement est conduit à une température supérieure de plus de 2,0 °C à $T_{3000\text{-}1}$ et est alimenté avec le mélange réactionnel obtenu dans le réacteur immédiatement précédent ;

(B) Isolement des di- et polyamines de la série du diphénylméthane à partir du mélange réactionnel (8-i) obtenu de l'étape (A-II) dans le dernier réacteur (3000-i), par au moins
(B-I) addition d'un excès stœchiométrique, par rapport à la quantité utilisée au total de catalyseur acide (7), de base (9) au mélange réactionnel (8-i) obtenu dans le dernier réacteur (3000-i) dans l'étape (A-II), avec obtention d'un mélange réactionnel (10) ;
(B-II) fractionnement du mélange réactionnel (10) obtenu dans l'étape (B-I) en une phase organique (11), comprenant des di- et polyamines de la série du diphénylméthane et une phase aqueuse (12),
où
dans le cas d'une modification de la capacité de production d'un état initial A avec

- un débit massique $m_1$ d'aniline utilisée au total, dans l'état initial, de $m_1(A) \neq 0$,
- un débit massique $m_2$ de formaldéhyde utilisé au total, dans l'état initial, de $m_2(A) = X(A)\ .\ m_2(N)$, X(*A*)

désignant un nombre sans dimension > 0 et ≤ 1 et $m_2(N)$ désignant la charge nominale de l'unité de production (10 000),

• un rapport molaire n(1)/n(2) d'aniline (1) utilisée au total à formaldéhyde (2) utilisé au total, dans l'état initial, de n(1)/n(2) (*A*),

• un rapport molaire n(7)/n(1) de catalyseur acide utilisé au total à aniline utilisée au total, dans l'état initial, de n(7)/n(1) (*A*) et

• une teneur massique $\omega_{MMDA}$ en diamines de la série du diphénylméthane, par rapport à la masse totale de di- et polyamines de la série du diphénylméthane, de $\omega_{MMDA}$ (*A*)

en un état final E avec

• un débit massique $m_1$ d'aniline utilisée au total de, dans l'état final, $m_1(E) \neq 0$,

• un débit massique $m_2$ de formaldéhyde utilisé au total, dans l'état final, de $m_2(E) = X(E) . m_2(N)$, X(E) désignant un nombre sans dimension > 0 et ≤ 1,

• un rapport molaire n(1)/n(2) d'aniline (1) utilisée au total à formaldéhyde (2) utilisé au total, dans l'état final, de n(1)/n(2) (*E*),

• un rapport molaire n(7)/n(1) de catalyseur acide utilisé au total à aniline utilisée au total, dans l'état final, de n(7)/n(1) (*E*) et

• une teneur massique $\omega_{MMDA}$ en diamines de la série du diphénylméthane, par rapport à la masse totale de di- et polyamines de la série du diphénylméthane, qui est recherchée pour l'état final, de $\omega_{MMDA}$ (*E*)

d'une valeur $\Delta X = |X(E) - X(A)|$, où $\Delta X \geq 0{,}10$, le procédé comprenant au moins une modification ainsi définie de la capacité de production, cette modification de la capacité de production commençant à un instant $t_1$ et étant terminée à un instant $t_2$, en outre $\omega_{MMDA}$ étant modifiée de telle façon que la valeur $\omega_{MMDA}(E)$ recherchée pour l'état final est soit $\geq 1{,}05 . \omega_{MMDA}(A)$, soit $\leq 0{,}95 . \omega_{MMDA}(A)$,

et où il s'applique que :

0,90 . n(7)/n(1) (*A*) ≤ n(7)/n(1) *(E)* ≤ 1,10 . n(7)/n(1) (*A*) ;

**caractérisé en ce que** dans l'espace de temps de $t_1$ à $t_2$, l'état de transition *Ü*, avec un rapport molaire d'aniline (1) utilisée au total à formaldéhyde (2) utilisé au total de n(1)/n(2) (*Ü*) et un rapport molaire de catalyseur acide utilisé au total à aniline utilisée au total de n(7)/n(1) (*Ü*)

(i) on règle la température dans le premier réacteur (3000-1), dans le sens de l'écoulement, à partir de l'étape (A-II), à une valeur qui s'écarte d'au maximum 10,0 °C de la température dans ce réacteur pendant l'état initial *A ;*

(ii-1) dans le cas de $m_2(E) > m_2(A)$, on augmente de plus de 2,0 °C, en comparaison de l'état initial *A*, la température dans au moins l'un des réacteurs suivants dans le sens de l'écoulement (3000-2, ..., 3000-i) de manière que la température finale recherchée soit présente au plus tard à l'instant $t_2$ et on maintient constante dans tous les réacteurs (3000-2, ..., 3000-i), dans lesquels la température n'est pas augmentée, la température dans la plage d'une amplitude de variation de ± 2,0 °C ;

(ii-2) dans le cas de $m_2(E) < m_2(A)$, on abaisse de plus de 2,0 °C, en comparaison de l'état initial *A*, la température dans au moins l'un des réacteurs suivants dans le sens de l'écoulement (3000-2, ..., 3000-i) de manière que la température finale recherchée soit présente au plus tard à l'instant $t_2$ et on maintient constante dans tous les réacteurs (3000-2, ..., 3000-i), dans lesquels la température n'est pas abaissée, la température dans la plage d'une amplitude de variation de ± 2,0 °C ;

(iii-1) dans le cas de $\omega_{MMDA}(E) \geq 1{,}05 . \omega_{MMDA}(A)$, on ajuste n(1)/n(2) (*Ü*) de manière à avoir à l'instant $t_2$ :
1,05 . n(1)/n(2) (*A*) ≤ n(1)/n(2) (*Ü*) ≤ 2,00 . n(1)/n(2) (*A*) ;
en ayant toujours pendant tout l'état de transition avant que soit atteint l'instant $t_2$ :
0,80 . n(1)/n(2) (*A*) ≤ n(1)/n(2) (*Ü*) ≤ 2,50 . n(1)/n(2) (*A*) ;

(iii-2) dans le cas de $\omega_{MMDA}(E) \leq 0{,}95 . \omega_{MMDA}(A)$, on ajuste n(1)/n(2) (*Ü*) de manière à avoir à l'instant $t_2$ :
0,50 . n(1)/n(2) (*A*) ≤ n(1)/n(2) (*Ü*) ≤ 0,95 . n(1)/n(2) (*A*) ;
en ayant toujours pendant tout l'état de transition avant que soit atteint l'instant $t_2$ :
0,40 . n(1)/n(2) (*A*) ≤ n(1)/n(2) (*Ü*) ≤ 2,50 . n(1)/n(2) (*A*).

**2.** Procédé selon la revendication 1, dans lequel la température dans les réacteurs de la cascade de réacteurs 3000 augmente du réacteur 3000-1 au réacteur 3000-i dans tous les états de fonctionnement (*A*, *Ü*, *E*).

**3.** Procédé selon l'une quelconque des revendications 1 et 2, dans lequel on règle toujours

• $T_{3000-1}$ à une valeur dans la plage de 25,0 °C à 65,0 °C et

• la température dans tous les réacteurs suivants dans le sens de l'écoulement (3000-2, ..., 3000-i) dans chaque cas à une valeur dans la plage de 35,0 °C à 200,0 °C.

**4.** Procédé selon la revendication 3, dans lequel on règle toujours

- $T_{3000-1}$ à une valeur dans la plage de 30,0 °C à 60,0 °C et
- la température dans tous les réacteurs suivants dans le sens de l'écoulement (3000-2, ..., 3000-i) dans chaque cas à une valeur dans la plage de 50,0 °C à 180,0 °C.

**5.** Procédé selon l'une quelconque des revendications précédentes, dans lequel le catalyseur acide (7) est un acide minéral.

**6.** Procédé selon l'une quelconque des revendications précédentes, dans lequel l'étape (B) comprend en outre :

(B-III) le lavage de la phase organique (11) avec du liquide de lavage (13) ;
(B-IV) le fractionnement du mélange (14) obtenu dans l'étape (B-III) en une phase organique (16) comprenant des di- et polyamines de la série du diphénylméthane et une phase aqueuse (15) ;
(B-V) la distillation de la phase organique (16) provenant de l'étape (B-IV) avec obtention des di- et polyamines de la série du diphénylméthane (18), avec séparation d'un courant (17) contenant de l'eau et de l'aniline.

**7.** Procédé selon la revendication 6, qui comprend en outre :
(C) le recyclage du courant (17), éventuellement après retraitement, dans l'étape (A-I) et/ou, si dans l'étape (A-II) on effectue l'addition optionnelle d'aniline supplémentaire (1), dans l'étape (A-II).

**8.** Procédé selon l'une quelconque des revendications précédentes, dans lequel, dans le cas de $\omega_{MMDA}(E) \geq 1,05 . \omega_{MMDA}(A)$, on abaisse $m_2(E)$ par rapport à $m_2(A)$.

**9.** Procédé selon l'une quelconque des revendications précédentes, dans lequel, dans le cas de $\omega_{MMDA}(E) \leq 0,95 . \omega_{MMDA}(A)$, on augmente $m_2(E)$ par rapport à $m_2(A)$.

**10.** Procédé selon l'une quelconque des revendications précédentes, dans lequel on ajuste le rapport molaire d'aniline (1) utilisée au total à formaldéhyde (2) utilisé au total, n(1)/n(2), dans tous les états de fonctionnement (*A*, *Ü*, *E*) à une valeur de 1,6 à 20.

**11.** Procédé selon l'une quelconque des revendications précédentes, dans lequel on maintient pendant la durée de la production avec le débit massique de formaldéhyde $m_2(E)$ les valeurs de n(1)/n(2), n(7)/n(1) et de la température d'un réacteur respectif j de la cascade de réacteurs (3000), $T_{3000-j}$, qui sont chaque fois présentes à l'instant $t_2$.

**12.** Procédé selon l'une quelconque des revendications précédentes, dans lequel pendant tout l'état de transition *Ü* avant que soit atteint l'instant $t_2$ il s'applique pour le rapport molaire de catalyseur acide utilisé au total à aniline utilisée au total n(7)/n(1) (*Ü*) :
$0,85 . n(7)/n(1) (A) \leq n(7)/n(1) (Ü) \leq 1,15 . n(7)/n(1) (A)$.

**13.** Procédé selon la revendication 10, dans lequel à l'instant $t_2$ il s'applique que :

$$n(7)/n(1)(E) = n(7)/n(1)(A).$$

**14.** Procédé selon l'une quelconque des revendications précédentes, dans lequel on ajuste jusqu'à l'instant $t_2$ la valeur finale recherchée du rapport molaire d'aniline utilisée au total à formaldéhyde utilisé au total, n(1)/n(2)(*E*) par adaptation continue de la valeur de $m_2$ et d'au moins l'une des valeurs de $m_1$ et/ou $m_7$.

**15.** Procédé selon l'une quelconque des revendications précédentes, dans lequel l'espace de temps de $t_1$ à $t_2$ dure de 1,00 minute à 120 minutes.

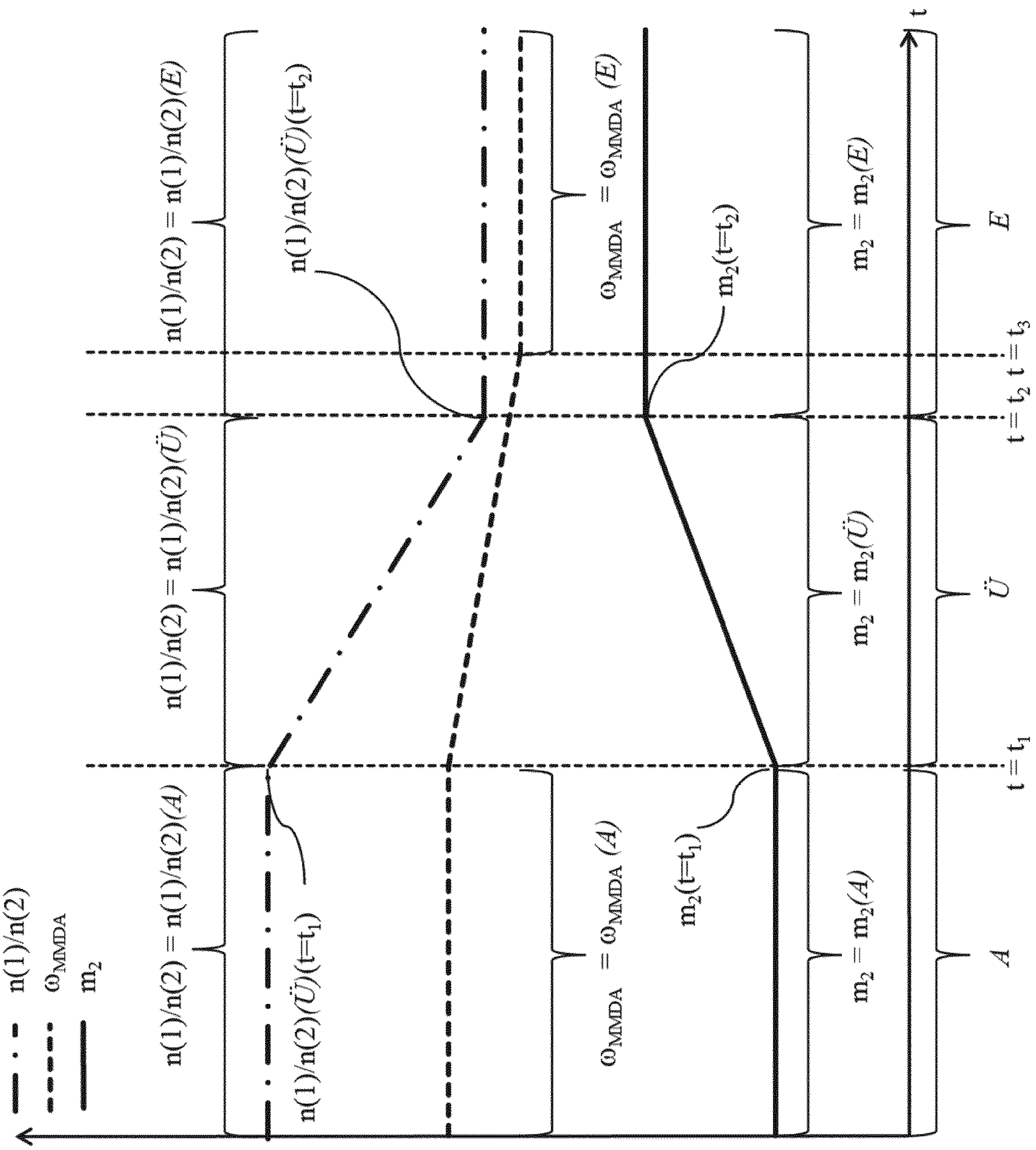

n(1)/n(2)
$\omega_{MMDA}$
$m_2$
n(1)/n(2) = n(1)/n(2)(A)
n(1)/n(2) = n(1)/n(2)(Ü)
n(1)/n(2) = n(1)/n(2)(E)
n(1)/n(2)(Ü)($t=t_1$)
n(1)/n(2)(Ü)($t=t_2$)
$\omega_{MMDA} = \omega_{MMDA}$ (A)
$\omega_{MMDA} = \omega_{MMDA}$ (E)
$m_2(t=t_1)$
$m_2(t=t_2)$
$m_2 = m_2(A)$
$m_2 = m_2(Ü)$
$m_2 = m_2(E)$
A
Ü
E
$t = t_1$
$t = t_2$
$t = t_3$
t


FIG. 1a

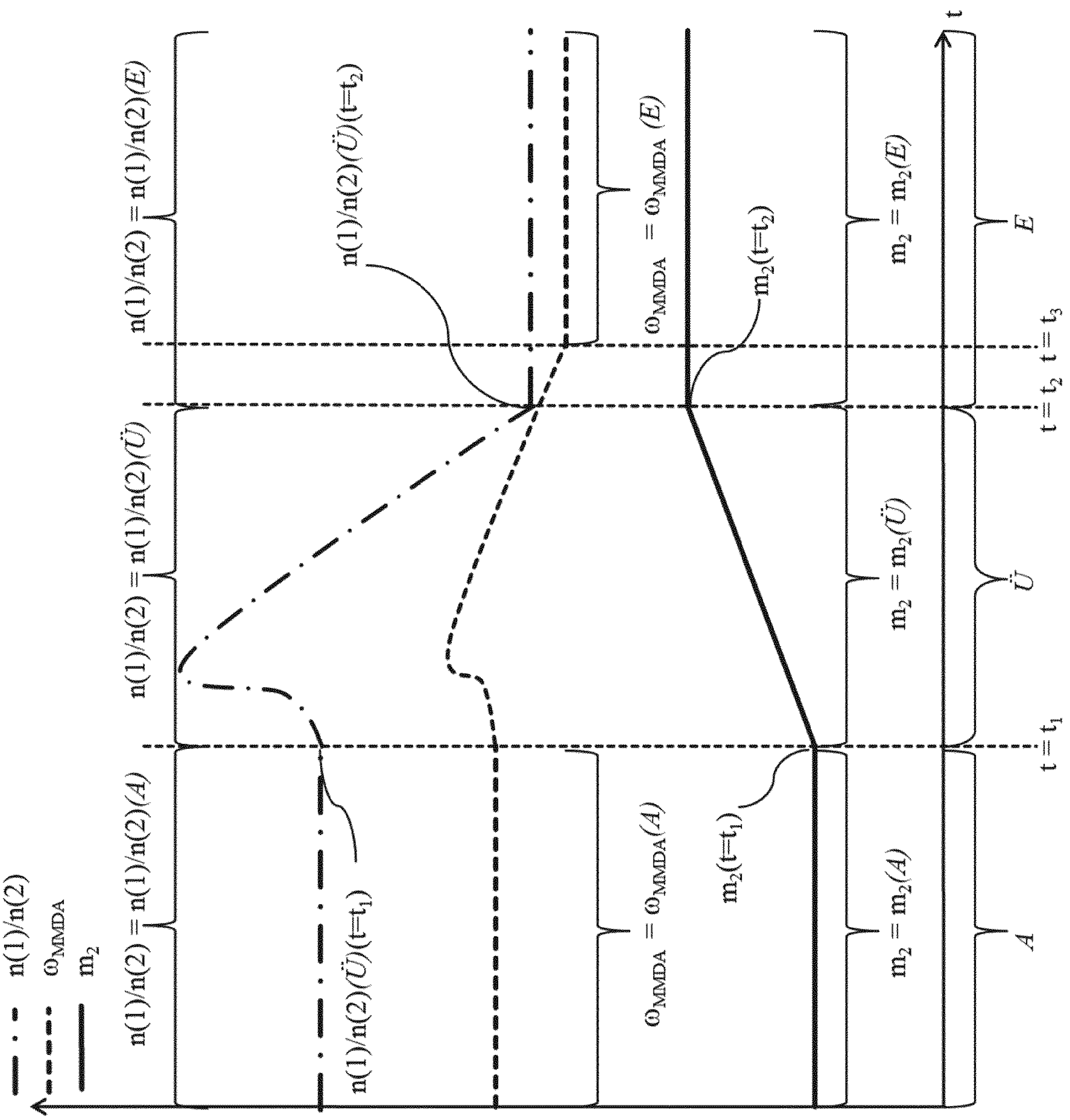
n(1)/n(2)
ωMMDA
m2
n(1)/n(2) = n(1)/n(2)(A)
n(1)/n(2) = n(1)/n(2)(Ü)
n(1)/n(2) = n(1)/n(2)(E)
n(1)/n(2)(Ü)(t=t1)
n(1)/n(2)(Ü)(t=t2)
ωMMDA = ωMMDA(A)
ωMMDA = ωMMDA (E)
m2(t=t1)
m2(t=t2)
m2 = m2(A)
m2 = m2(Ü)
m2 = m2(E)
A
Ü
E
t = t1
t = t2
t = t3
t

FIG. 1b

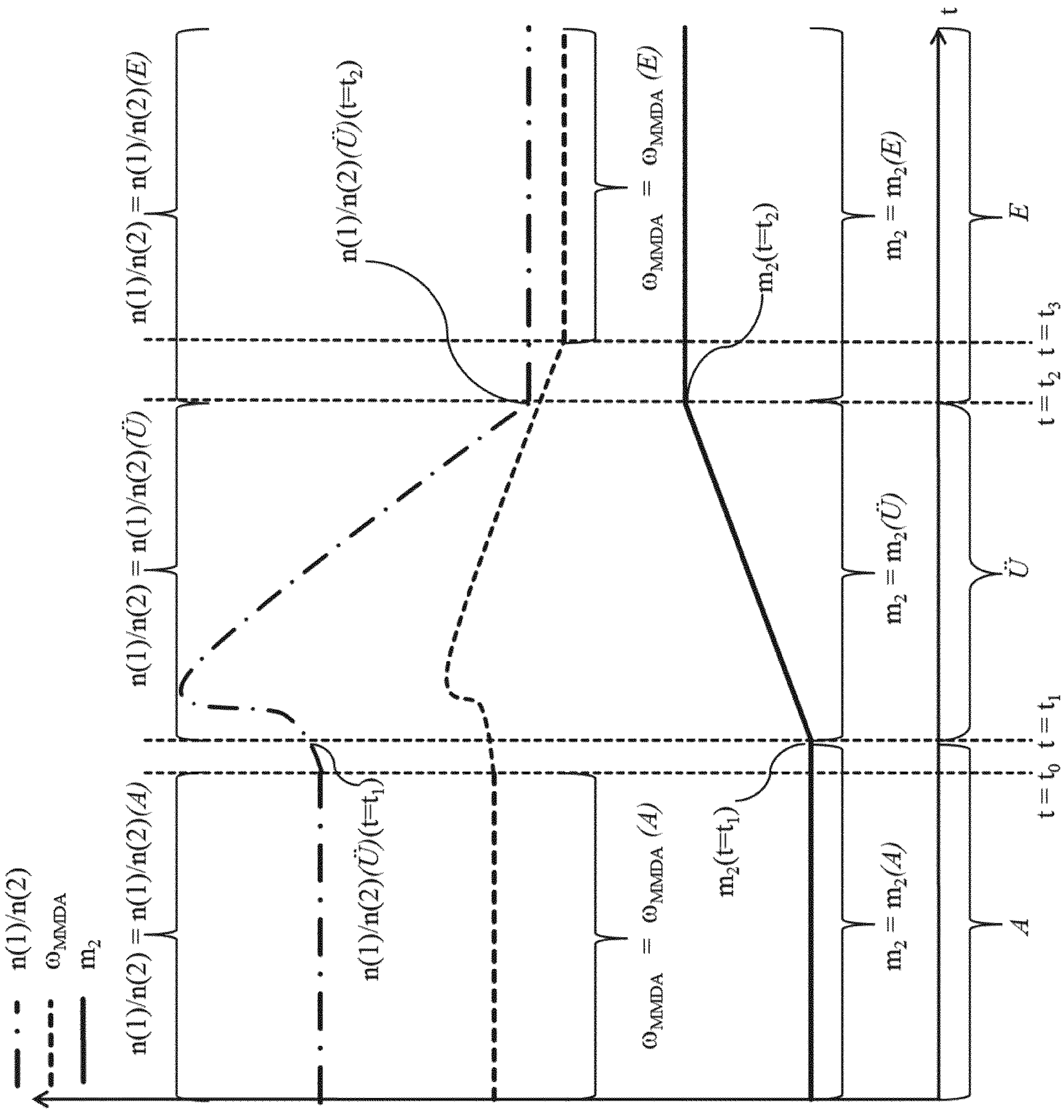

n(1)/n(2)
ω_MMDA
m_2
n(1)/n(2) = n(1)/n(2)(A)
n(1)/n(2) = n(1)/n(2)(Ü)
n(1)/n(2) = n(1)/n(2)(E)
n(1)/n(2)(Ü)(t=t_1)
n(1)/n(2)(Ü)(t=t_2)
ω_MMDA = ω_MMDA (A)
ω_MMDA = ω_MMDA (E)
m_2(t=t_1)
m_2(t=t_2)
m_2 = m_2(A)
m_2 = m_2(Ü)
m_2 = m_2(E)
A
Ü
E
t = t_0
t = t_1
t = t_2
t = t_3
t


FIG. 1c

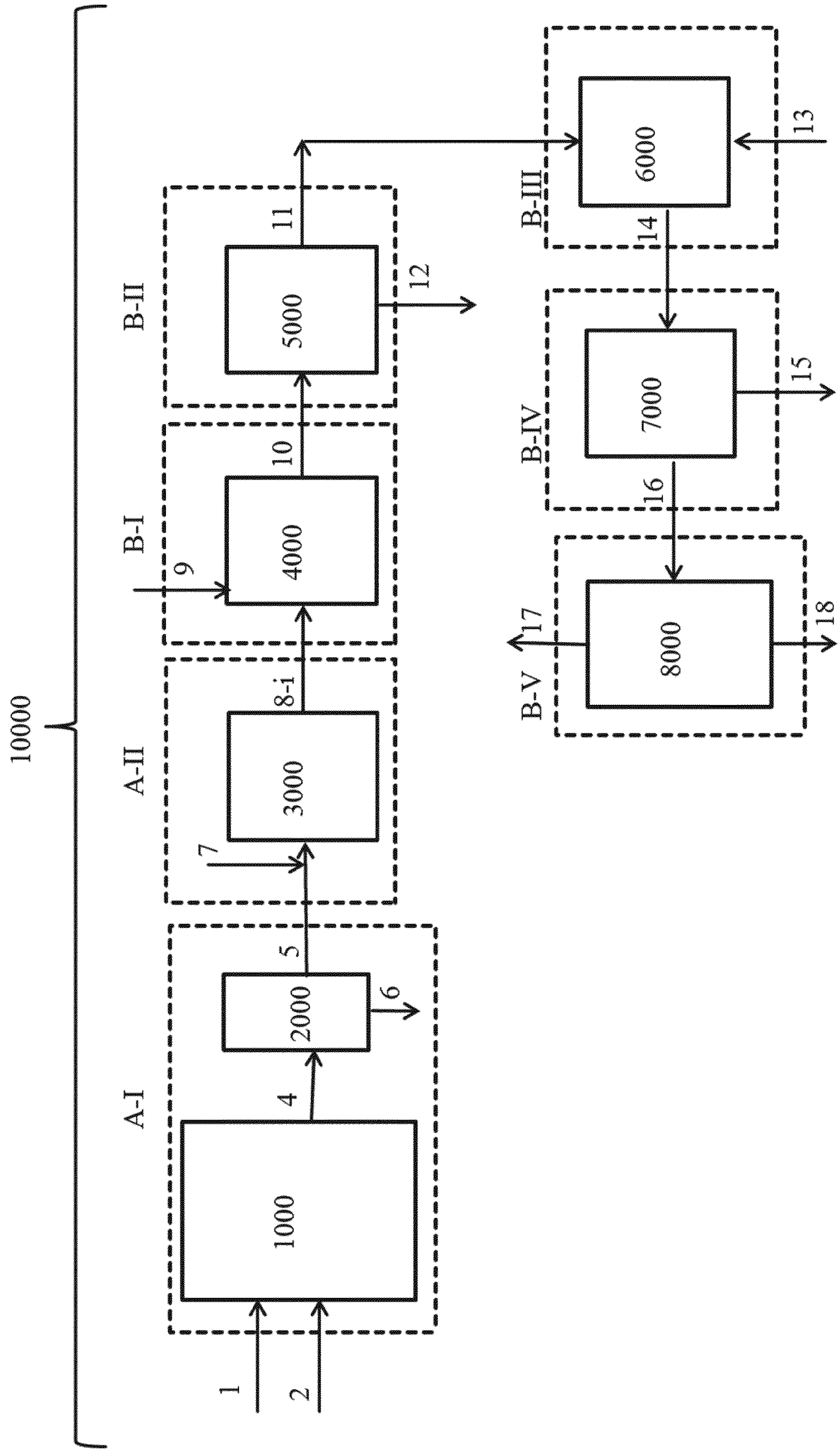
10000
A-I
1000
2000
1
2
4
5
6
A-II
3000
7
8-i
B-I
4000
9
10
B-II
5000
11
12
B-III
6000
13
14
B-IV
7000
15
16
B-V
8000
17
18

FIG. 2

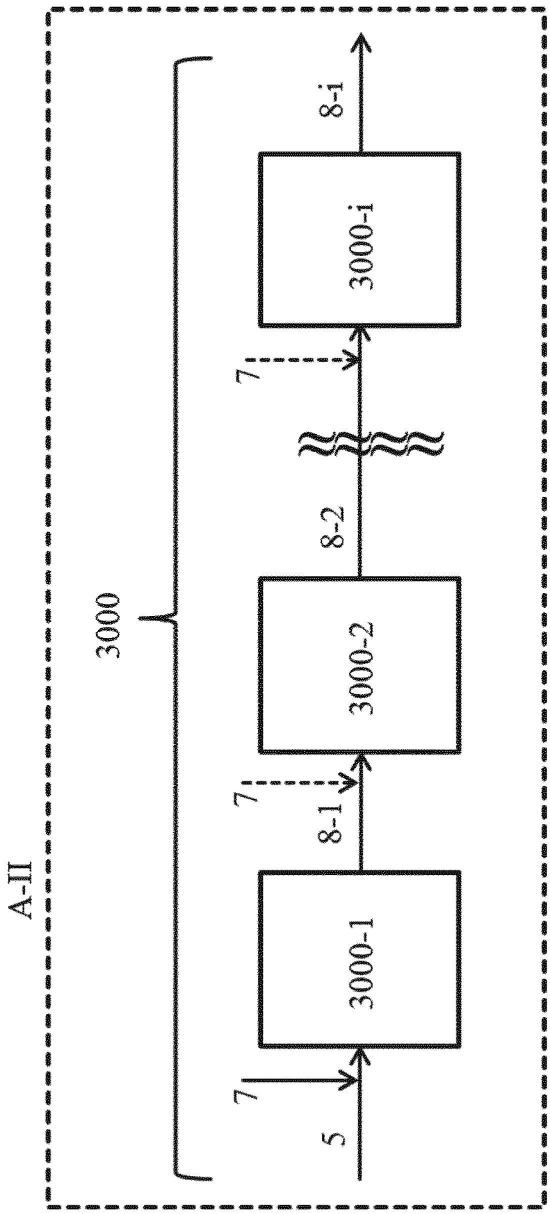


FIG. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1616890 A
- US 5286760 A
- EP 0451442 A
- WO 9940059 A
- EP 1652835 A
- EP 2103595 A
- WO 2014173856 A1
- WO 2015197527 A1
- WO 2015197519 A1
- WO 2015197520 A1
- EP 1257522 B1
- EP 2103595 A1
- EP 1813598 B1
- EP 1616890 A1
- EP 1270544 A1
- EP 1652835 A1
- DE 2549890 A
- EP 1813597 B1
- DE 844896 A
- DE 19817691 A


### In der Beschreibung aufgeführte Nicht-Patentliteratur


- **H. J. TWITCHETT.** *Chem. Soc. Rev,* 1974, vol. 3 (2), 223
- Mass-Transfer Operations. McGraw-Hill Book Co, 1980, 477-541
- Liquid-Liquid Extraction. **E. MÜLLER et al.** Ullmann's Encyclopedia of Industrial Chemistry. Wiley-VCH Verlag GmbH & Co. KGaA, 2012, vol. 21, 272-274
- Kirk-Othmer Encyclopedia of Chemical Technology. 15. Juni 2007, 22-23
- *Aminalabscheider,* 2000